(19) Europäisches Patentamt
European Patent Office
Office européen des brevets

(11) EP 1 676 565 A1

(12) DEMANDE DE BREVET EUROPEEN

(43) Date de publication:
**05.07.2006 Bulletin 2006/27**

(21) Numéro de dépôt: **05292449.5**

(22) Date de dépôt: **18.11.2005**

(51) Int Cl.:
***A61K 8/39*** (2006.01) ***A61K 8/81*** (2006.01)
***A61Q 1/06*** (2006.01) ***A61Q 19/00*** (2006.01)

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **30.12.2004 FR 0453268**

(71) Demandeur: **L'ORÉAL**
**75008 Paris (FR)**

(72) Inventeurs:
- **Ricard, Audrey**
  **75012 Paris (FR)**
- **Lebre, Caroline**
  **94320 Thiais (FR)**

(74) Mandataire: **Boulard, Denis**
**L'OREAL - D.I.P.I.**
**25-29 Quai Aulagnier**
**92600 Asnières (FR)**

(54) **Composition cosmétique contenant un ester d'alcool alcoxylé et un polymère filmogène**

(57) La présente demande concerne une composition comprenant au moins un ester d'alcool alcoxylé et d'acide carboxylique et au moins un polymère filmogène solide et non critallin.

L'ester d'alcool alcoxylé peut être notamment l'Octyldodécyl PPG-3 Myristyl Ether Dimer Dilinoléate.

Cette composition peut être utilisée en tant que produit de soin et/ou de maquillage des matières kératiniques, notamment de la peau, des lèvres et/ou des phanères.

EP 1 676 565 A1

## Description

La présente invention se rapporte à une composition cosmétique, notamment une composition cosmétique de maquillage ou de soin de la peau du visage ou du corps humain, du cuir chevelu, des lèvres ou des phanères, comme les cheveux, les cils, les sourcils ou les ongles.

La composition de l'invention peut en particulier constituer un produit de maquillage des lèvres, du corps ou des phanères pouvant être doté en outre de propriétés de soin. La composition de l'invention peut constituer notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

La composition cosmétique selon l'invention présente avantageusement une tenue de la couleur très satisfaisante tout en étant brillante et confortable.

La tenue est particulièrement recherchée par les utilisateurs de produits cosmétiques. Des rouges à lèvres de tenue satisfaisante sont généralement des sticks contenant des huiles esters de faible poids moléculaire ou des huiles silicones. Afin d'augmenter la tenue de ces produits, les derniers concepts de formule s'appuient sur l'association d'une phase « brillante » et d'une phase « couleur et confort » rendues compatibles par un solvant volatil. Lors de l'application du produit sur les lèvres, le volatile s'évapore puis un phénomène de ségrégation se produit. Ce concept reste toutefois limité pour améliorer la tenue et le confort. En effet, le domaine de compatibilité des phases reste restreint et le taux d'isododécane ne peut être augmenter au détriment de la dureté des sticks et surtout du confort.

On a proposé des formules contenant des copolymères acrylates/acryliques dispersés dans un solvant volatile tel que l'isododécane. Ces polymères forment un film sur les lèvres après évaporation du solvant. Il faut néanmoins de très fort taux de volatiles dans ces formules pour assurer la tenue de la composition cosmétique sur les matières kératiniques, au détriment du confort du dépôt sur les matières kératiniques.

Il a été trouvé, de façon surprenante, qu'un ester alcoxylé en association avec certains filmogènes permet d'obtenir des formules dont la brillance est élevée et dont le confort est équivalent à une formule classique tout en améliorant de façon importante la tenue de la couleur par rapport aux formules connues.

Certains esters alcoxylés ont déjà été utilisés dans des compositions cosmétiques.

Ainsi, la demande US 2002/0192249 décrit des compositions cosmétiques comprenant un ester d'acide monocarboxylique comprenant de 4 à 24 atomes de carbone, et d'alcool comprenant un groupe polypropoxyle et une chaîne alkyle comportant de 2 à 24 atomes de carbone.
Les préparations peuvent comporter, outre l'ester alcoxylé, de l'huile minérale ou de l'huile de paraffine. Ce document décrit également une composition anhydre contenant cet ester et un agent filmogène, et une composition contenant du PPG-3 Myristyl Ether Néoheptanoate. Plus précisément, le document donne des compositions de rouges à lèvres contenant cet ester polypropoxylé en association avec du polyisobutène hydrogéné ; des compositions de fond de teint en crème en émulsion contenant cet ester ; des compositions de filtres solaires en émulsion contenant du laurate d'hexyle, du palmitate d'octyle, et du palmitate de cétyle en association avec cet ester.

Le document US 5.693.316 divulgue des compositions cosmétiques contenant un ester gras alcoxylé obtenu à partir d'un acide dicarboxylique ayant de 2 à 22 atomes de carbone, en particulier l'acide maléique, et d'un excès stoechiométrique de un ou plusieurs alcools gras polyalcoxylés comportant une chaîne alkyle comprenant de 14 à 22 atomes de carbone et un groupe polyalcoxyle. Les préparations peuvent comporter de l'huile minérale ou de l'huile de paraffine comme deuxième émollient. Ce document décrit également une composition anhydre contenant cet ester et un agent filmogène. L'ester alcoxylé est en particulier le Di-PPG-3 Myristyl maléate.

Le document US 6.476.254 divulgue des compositions cosmétiques contenant un ester d'acide dicarboxylique comprenant de 4 à 12 atomes de carbone et d'alcool gras polyalcoxylé dont la partie non alcoxylée comprend de 8 à 36 atomes de carbones. L'ester peut être le Di-PPG-3 Myristyl adipate. La composition peut être anhydre. Elle peut contenir de l'huile minérale ou de l'huile de paraffine.

La demande WO 2003/013439 a pour objet un ester d'acide dicarboxylique en C3-C21 ou tricarboxylique aliphatique en C4-C22, notamment de C3 à C9, et d'alcool gras polyalcoxylé comprenant un radical alkyle en C6-C30, notamment en C18-C22. Ce document décrit une composition cosmétique qui peut contenir de la vaseline, de l'huile minérale, des esters d'acides carboxylique aliphatiques et d'alcools aliphatiques comprenant de 18 à 40 atomes de carbone, un agent filmogène, ou un alcool gras tel que l'alcool cétylique.

Les documents US 5.302.377, US 5.455.025 et US 5.597.555 divulguent des compositions cosmétiques contenant un ester gras alcoxylé d'acide tricarboxylique, en particulier l'acide citrique, avec un excès stoechiométrique de un ou plusieurs alcools gras polyalcoxylés ayant des propriétés émollientes pour des préparations topiques. Les préparations contiennent une huile minérale comme deuxième émollient. Ce document décrit également l'association de cet ester avec un agent filmogène. L'ester est en particulier le Tri-PPG-3 Myristyl citrate.

Le document WO 2004 052076 décrit des compositions cosmétiques contenant des esters mixtes d'alcools polyalcoxylés et d'alcools monohydriques avec des acides polycarboxyliques, notamment des acides dicarboxyliques. Ces compositions peuvent contenir un deuxième agent émollient tel que l'huile minérale ou la vaseline. Les esters

mixtes décrits peuvent être formulés en association avec un composé filmogène.

La présente invention a pour premier objet une composition cosmétique comprenant au moins un ester d'alcool alcoxylé et d'acide carboxylique et au moins un polymère filmogène non cristallin solide à température ambiante.

Par solide à température ambiante, on entend un polymère qui ne s'écoule pas sous son propre poids. Un polymère sous la forme d'une poudre est solide au sens de l'invention. En ce qui concerne un polymère visqueux ou très visqueux, la capacité à s'écouler sous son propre poids peut en particulier être évaluée en plaçant 20g du polymère sur un support, et à tracer le contour du dépôt immédiatement après avec un feutre noir. L'échantillon est ainsi laissé pendant environ 2 heures à une température régulée de 25 °C. Au terme de ce délai, aucun écoulement en dehors de la zone de dépôt n'est observé à l'oeil nu.

La présente invention a pour deuxième objet une composition cosmétique comprenant au moins un ester d'alcool alcoxylé et d'acide carboxylique et au moins un polymère filmogène non cristallin choisi parmi les polymères et copolymères vinyliques notamment acryliques et éthyléniques, les polymères et copolymères uréthanes, les polymères et copolymères polyesters, les polymères et copolymères polyamides, notamment les polyamides siliconés, les polymères et copolymères polyurées, les polymères et copolymères cellulosiques comme la nitrocellulose, les polymères et copolymères siliconés.

De façon avantageuse la composition est apte à former un dépôt dont la tenue est supérieure ou égale à 30%.

De façon avantageuse, le polymère filmogène est tel, que lorsqu'il est en quantité suffisante dans la composition, celle-ci est apte à former un dépôt dont la tenue est supérieure ou égale à 30%.

TENUE

Avantageusement, la composition est apte à former un dépôt ayant un indice de tenue supérieur ou égal à 30 %, de préférence supérieur ou égal à 40 %, de préférence supérieur ou égal à 45 %, de préférence supérieur ou égal à 50 %.

L'indice de tenue du dépôt obtenu avec la composition selon l'invention peut être déterminé selon le protocole de mesure décrit ci-après.

On prépare un support (rectangle de 40 mm X 70 mm) constitué d'un revêtement acrylique (adhésif acrylique hypoallergénique sur film polyéthylène vendu sous la dénomination BLENDERME ref FH5000-55113 par la société 3M Santé) collé sur une couche de mousse de polyéthylène adhésif sur la face opposée à celle sur laquelle est fixé le sparadrap (couche de mousse vendue sous la dénomination RE40X70EP3 de la société JOINT TECHNIQUE LYONNAIS IND).

On mesure à l'aide d'un colorimètre MINOLTA CR 300 la couleur $L^*_0a^*_0b^*_0$ du support , côté face revêtement acrylique.

On préchauffe le support ainsi préparé sur une plaque chauffante maintenue à la température de 40 °C pour que la surface du support soit maintenue à une température de 33 °C ± 1 °C.

Tout en laissant le support sur la plaque chauffante, on applique la composition sur toute la surface non adhésive du support (c'est-à-dire sur la surface du revêtement acrylique) en l'étalant à l'aide d'un pinceau pour obtenir un dépôt de la composition d'environ 15 µm puis on laisse sécher pendant 10 minutes.

Après séchage, on mesure la couleur L*a*b* du film ainsi obtenu.

On détermine alors la différence de couleur ΔE1 entre la couleur du film par rapport à la couleur du support nu par la relation suivante.

$$\Delta E1 = \sqrt{(L^*-L_o^*)^2 + (a^* - a_o^*)^2 + (b^* - b_o^*)^2}$$

Le support est ensuite collé par sa face adhésive (face adhésive de la couche de mousse) sur une enclume d'un diamètre de 20 mm et munie d'un pas de vis. Une éprouvette de l'ensemble support/dépôt est ensuite découpée à l'aide d'un emporte- pièce d'un diamètre de 18 mm. L'enclume est ensuite vissée sur une presse (STATIF MANUEL IMADA SV-2 de la société SOMECO) équipée d'un dynanomètre (IMADA DPS-20 de la société SOMECO).

Sur un papier blanc pour photocopieuse de grammage 80g/m2 , on dessine une bande de 33 mm de largeur et 29,7 cm de longueur, on trace un premier trait à 2 cm du bord de la feuille, puis un deuxième trait à 5 cm du bord de la feuille, les premier et deuxième traits délimitant ainsi une case sur la bande ; puis on dispose une première marque et une deuxième marque situées dans la bande respectivement aux repères 8 cm et 16 cm du deuxième trait . On place sur la première marque 20 µl d'eau et sur la deuxième marque 10 µl d'huile de tournesol raffinée (vendue par la société

LESIEUR).

Le papier blanc est placé sur le socle de la presse puis on presse l'éprouvette placée sur la case de la bande de papier à une pression d'environ 300 g/cm$^2$ exercée pendant 30 secondes. Puis on relève la presse et on place à nouveau l'éprouvette juste après le deuxième trait (donc à côté de la case), on effectue à nouveau une pression d'environ 300 g/cm$^2$ et on déplace, de manière rectiligne dès le contact effectué, le papier avec une vitesse de 1 cm/s, sur toute la longueur de la bande de telle sorte que l'éprouvette traverse les dépôts d'eau et d'huile.

Après retrait de l'éprouvette, une partie du dépôt a transféré sur le papier. On mesure alors la couleur L*', a*', b*' du dépôt resté sur l'éprouvette.

On détermine alors la différence de couleur ΔE2 entre la couleur du dépôt resté sur l'éprouvette par rapport à la couleur du support nu par la relation suivante.

$$\Delta E2 = \sqrt{(L^{*\prime}-L_o^*)^2 + (a^{*\prime} - a_o^*)^2 + (b^{*\prime} - b_o^*)^2}$$

L'indice de tenue de la composition, exprimée en pourcentage, est égal au rapport 100 X ΔE2/ΔE1

La mesure est effectuée sur 6 supports à la suite et l'indice de tenue correspond à la moyenne des 6 mesures obtenues avec les 6 supports.

L'invention a également pour objet un procédé cosmétique pour conférer à un film de composition cosmétique des propriétés de brillance, de tenue et de confort, consistant à introduire dans ladite composition au moins un ester d'alcool alcoxylé et d'acide carboxylique et au moins un polymère filmogène tel que défini précédemment selon le premier ou le deuxième objet de l'invention.

L'invention a encore pour objet l'utilisation de l'association d'au moins un ester d'alcool alcoxylé et d'acide carboxylique et d'au moins un polymère filmogène tel que défini précédemment, pour obtenir une composition cosmétique dotée de propriétés de brillance, de tenue et de confort.

L'invention a encore pour objet un procédé de soin et/ou de maquillage des matières kératiniques qui consiste à appliquer sur les matières kératiniques une composition comprenant au moins un ester d'alcool alcoxylé et d'acide carboxylique et au moins un polymère filmogène tel que défini précédemment, ladite composition étant apte à former un dépôt ayant un indice de tenue supérieur ou égal à 30 %.

On entend par alcool alcoxylé, un composé hydrocarboné comprenant au moins une fonction -OH, de préférence une seule, et au moins un groupe

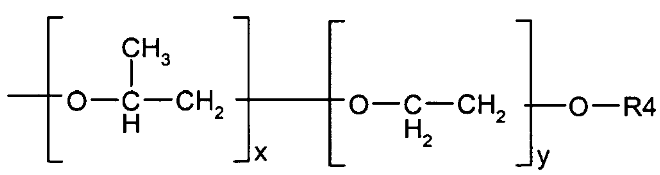

dans laquelle x et y sont indépendamment des entiers de 0 à 40 inclus, et la somme de x et y est comprise entre 1 et 80 inclus, et $R_4$ est un motif hydrocarboné aliphatique ou aromatique, saturé ou insaturé, substitué ou non, contenant de 1 à 36 atomes de carbone, notamment de 4 à 36 atomes de carbone.

L'alcool alcoxylé est de préférence un alcool polyalcoxylé dont le groupe de formule ci-dessus est tel que x et y sont indépendamment des entiers de 0 à 40 inclus, et la somme de x et y est comprise entre 2 et 80 inclus. x et y sont de préférence indépendamment des entiers de 0 à 30 inclus, et la somme de x et y est comprise entre 2 et 30 inclus.

La formule précédente illustre schématiquement tous les motifs éthoxy dans un premier groupe et tous les motifs propoxy dans un autre groupe. En fait, ces motifs peuvent être placés dans n'importe quel ordre, de façon aléatoire, en blocs et sous forme de motifs alternés. A titre purement illustratif, les motifs éthoxy (E) et les motifs propoxy (P) de l'alcool alcoxylé peuvent être disposés de la façon suivante : EEEP, EEPE, EPEE, PEEE, EEEPEPPPE, PEPPPEEEEPE et selon des arrangements similaires.

## ESTER ALCOXYLE

La composition selon l'invention contient au moins un ester d'alcool alcoxylé et d'acide carboxylique, appelé par la suite ester alcoxylé, qui peut être choisi parmi

- les esters obtenus par réaction d'un acide monocarboxylique avec un alcool alcoxylé,
- les polyesters obtenus par réaction d'un acide polycarboxylique avec un excès stoechiométrique d'au moins un alcool alcoxylé par rapport au nombre de fonctions acides dudit acide,
- les polyesters dont une seule fonction ester est obtenue par réaction d'une fonction acide d'un acide polycarboxylique avec un alcool alcoxylé,
- les polyesters comprenant au moins une fonction ester obtenue par réaction d'une fonction acide d'un acide polycarboxylique avec un alcool alcoxylé, et au moins une fonction ester obtenue par réaction d'une autre fonction acide dudit acide polycarboxylique avec un alcool gras,
- et leurs mélanges.

**Ester alcoxylé d'acide monocarboxylique**

L'ester alcoxylé peut être choisi parmi les esters d'acide monocarboxylique et d'alcools gras alcoxylé, en particulier polyalcoxylé. En particulier, l'ester alcoxylé est choisi parmi les esters formés par la réaction d'un acide monocarboxylique aliphatique ou aromatique avec un excès stoechiométrique d'un alcool gras polyalcoxylé, par exemple un alcool polypropoxylé.

L'ester alcoxylé d'acide monocarboxylique peut être choisi parmi les monoesters polypropoxylés de formule développée suivante :

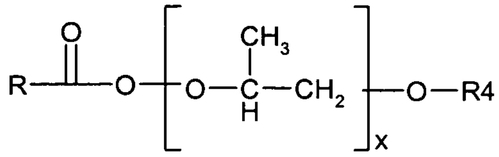

dans laquelle x est un entier de 2 à 40 inclus, de préférence de 3 à 30, notamment entre 3 et 10,
$R_4$ est un motif hydrocarboné aliphatique saturé ou insaturé, substitué ou non, contenant de 1 à 36 atomes de carbone, de préférence de 3 à 24 atomes de carbone, de préférence de 4 à 24 atomes de carbone et
RCOO correspond à un acide monocarboxylique RCOOH aliphatique ou aromatique.

RCOO peut être

- un reste d'acide monocarboxylique par exemple un acide de formule (R2R3R4C)COO dans laquelle $R_2$, $R_3$ et $R_4$ sont choisis indépendamment dans le groupe constitué de méthyle, d'éthyle, de propyle ou d'isopropyle ; ou
- un reste d'acide aromatique comprenant un cycle benzényle éventuellement substitué par OH ou $NH_2$ ou méthyle ou éthyle.

Les acides monocarboxyliques aliphatiques convenant à la préparation de l'ester alcoxylé peuvent comprendre de 4 à 24 atomes de carbone, notamment de 4 à 18 atomes de carbone. Des exemples d'acides monocarboxyliques aliphatiques incluent l'acide 2-éthylhexanoïque, l'acide caproïque, l'acide néopentanoïque, l'acide isostéarique, l'acide néoheptanoïque et l'acide oléique.

Des exemples d'acides monocarboxyliques aromatiques incluent l'acide benzoïque et l'acide p-aminobenzoïque.

Les alcools gras utilisés pour préparer les esters alcoxylés peuvent être saturés ou insaturés, substitués ou non, aliphatiques ou aromatiques, et peuvent être à chaîne droite ou à chaîne ramifiée. Ils peuvent avoir entre 6 et 24 atomes de carbone, notamment entre 12 et 14 atomes de carbone.

Par alcool gras, on entend un alcool aliphatique ayant au moins trois atomes de carbone. De préférence, l'alcool gras est constitué d'atomes de carbone, d'hydrogène et d'oxygène. Il peut être saturé ou comporter au moins une double liaison carbone carbone.
Un alcool gras peut notamment être un alcool obtenu par hydrolyse de graisses ou d'huiles végétales ou animales.

Les esters d'acide monocarboxylique et d'alcool gras polypropoxylé sont par exemple choisis parmi le PPG-3 Myristyl Ether Néoheptanoate commercialisé sous la référence Trivasperse, le PPG-4 Butyloctyl Ether Ethylhexanoate et leurs mélanges.

Ces esters peuvent être préparés selon l'enseignement du document US 2002/0192249 dont le contenu est incorporé dans la présente demande par référence.

**Polyesters mixtes alcoxylés**

En particulier, l'ester alcoxylé peut être choisi parmi les esters mixtes d'alcool alcoxylé et d'alcool monohydrique avec des acides polycarboxyliques, notamment des acides dicarboxyliques.

Notamment, l'ester alcoxylé est choisi parmi les esters mixtes d'alcool gras polyalcoxylé et d'alcool gras monohydrique avec des acides gras dicarboxyliques.

Par ester mixte on entend un ester obtenu par réaction d'un acide polycarboxylique avec au moins deux alcools différents.

Par acide gras, on entend un acide carboxylique aliphatique ayant au moins trois atomes de carbone. De préférence, l'acide gras est constitué d'atomes de carbone, d'hydrogène et d'oxygène. Il peut être saturé ou comporter au moins une double liaison carbone carbone.

Un acide gras peut notamment être un acide carboxylique obtenu par hydrolyse de graisses ou d'huiles végétales ou animales.

L'ester mixte d'alcool alcoxylé peut être notamment choisi parmi les composés de formule développée suivante :

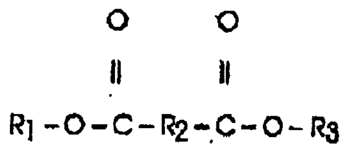

dans laquelle $R_1$ a la formule développée :

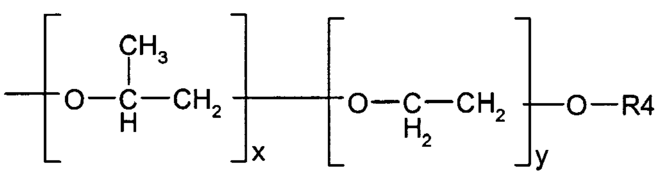

dans laquelle :

$R_4$ est un motif aliphatique saturé ou insaturé, substitué ou non, contenant de 4 à 24 atomes de carbone ;

x est un entier de 3 à 30 ; y est un entier de 3 à 30

$R_2$ est un motif aliphatique saturé ou insaturé, substitué ou non, contenant de 4 à 40 atomes de carbone ; et

$R_3$ est un motif aliphatique à chaîne droite ou à chaîne ramifiée, saturé ou insaturé, contenant de 4 à 32 atomes de carbone, notamment de 12 à 24 atomes de carbone.

Les exemples de composés correspondant à la formule générale précédente sont

- l'Octyldodécyl PPG-3 Myristyl Ether Dilinoléate commercialisé sous la référence LIQUIWAX polyEFA par la société ARCH CHEMICAL de formule

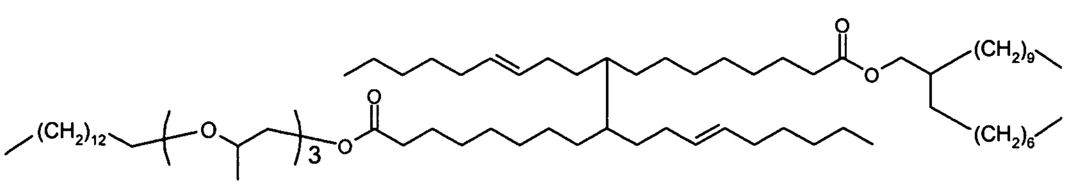

- le Stéaryl PPG-3 Myristyl Ether Dilinoléate commercialisé sous la référence liquiwax polylPL par la société ARCH

CHEMICAL, et
- l'Isostéaryl PPG-4 Butyloctyl Ether Dilinoléate.

Ces esters mixtes peuvent être produits par la réaction d'alcools gras alcoxylés et d'alcools gras monohydriques avec des acides gras dicarboxyliques.
De préférence, les alcools gras alcoxylés sont des alcools gras propoxylés et ont une longueur de chaîne carbonée comprise entre 4 et 24 atomes de carbone et un taux de propoxylation compris entre 3 et 30, et de façon tout à fait préférable compris entre 3 et 15 motifs d'oxyde de propylène. Les alcools gras propoxylés préférés sont l'alcool myristique et le butyloctanol.
L'acide dicarboxylique contient au moins deux groupes carboxyliques par molécule. Il peut notamment être représenté par la formule (I) suivante :

$$HOOC\text{-}(CH_2)_n\text{-}COOH \qquad (I)$$

dans laquelle n est un nombre entier de 1 à 16, de préférence de 3 à 16. A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-ndécaméthylènedicarboxylique, l'acide 1,12-dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.
L'acide dicarboxylique peut également être un dimère diacide. Un dimère diacide désigne un diacide obtenu par réaction de polymérisation, notamment de dimérisation, intermoléculaire d'au moins un acide mono-carboxylique insaturé. Ils dérivent en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.
A titre représentatif de ces acides gras insaturés, on peut notamment citer comme précédemment l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.
Selon une variante particulière, il s'agit plus particulièrement du dimère diacide dont dérive également le dimère diol à estérifier.
Plus particulièrement, il s'agit du dimère diacide obtenu par dimérisation de l'acide linoléique, éventuellement suivie d'une hydrogénation des liaisons carbone carbone. Le dimère diacide peut être sous forme saturée, c'est-à-dire ne comporter aucune double liaison carbone carbone. Selon un autre mode de réalisation, les éventuelles doubles liaisons carbone carbone du dimère diacide sont toutes ou en partie hydrogénées, après réaction d'estérification du dimère diacide avec le dimère diol.
Selon un mode de réalisation, le dimère diacide est un produit commercialisé constitué d'un acide dicarboxylique ayant environ 36 atomes de carbone. Ce produit contient également un acide trimérique et un acide monomère, dans des proportions qui dépendent du degré de pureté du produit. Classiquement, on trouve dans le commerce des produits dont la teneur en dimère diacide est supérieure à 70 % et d'autres dont la teneur en dimère diacide a été ajustée à 90 % ou plus.
On trouve également dans le commerce des dimères diacides et notamment des diacides dilinoléiques dont la stabilité vis-à-vis de l'oxydation a été améliorée par hydrogénation des doubles liaisons restant après la réaction de dimérisation. Dans la présente invention, on peut utiliser tout dimère diacide disponible actuellement dans le commerce.
Les alcools gras monohydriques peuvent être avoir une longueur de chaîne carbonée comprise entre 12 et 24. Les alcools gras monohydriques préférés sont l'octyldodécanol et l'alcool isostéarique.
Des exemples de préparation des esters décrits précédemment sont donnés dans la demande WO 2004 052076 dont le contenu est incorporé par référence dans la présente demande.

Polyesters alcoxylés

L'ester alcoxylé peut être obtenu par estérification d'un acide polycarboxylique par au moins deux alcools alcoxylés, lesquels peuvent être identique ou différents, de manière à former un ester.
L'ester peut notamment être choisi parmi les esters de formule :

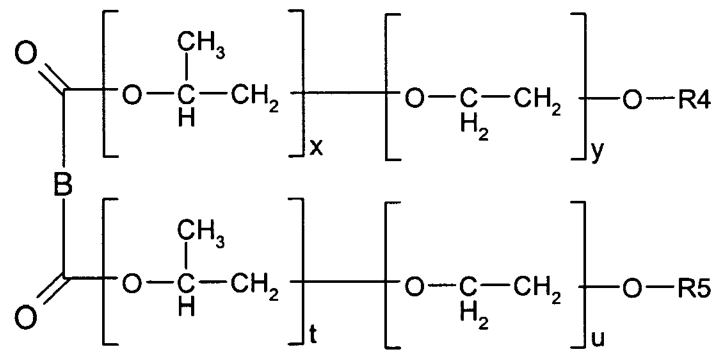

dans laquelle

-OOC-B-COO- est le reste d'un acide dicarboxylique tel que décrit précédemment, ayant notamment de 2 à 40 atomes de carbone, saturé ou insaturé, substitué ou non substitué,
x et y sont indépendamment des entiers de 0 à 40 inclus, et la somme de x et y est comprise entre 1 et 80 inclus, de préférence entre 2 et 80 inclus,
t et u sont indépendamment des entiers de 0 à 40 inclus, et la somme de t et u est comprise entre 1 et 80 inclus, de préférence entre 2 et 80 inclus,
$R_4$ et $R_5$ sont indépendamment l'un de l'autre des motifs hydrocarbonés aliphatiques ou aromatiques, saturés ou insaturés, substitués ou non, contenant de 4 à 36 atomes de carbone.

Selon un mode de mise en oeuvre, $R_4$ et $R_5$ peuvent être identiques ou différents et comportent notamment de 10 à 22 atomes de carbone et peuvent être saturés ou insaturés, substitués ou non substitués,
**[0066]** Selon un mode de mise en oeuvre, y vaut de 1 à 40 et x vaut de 0 à 30 à la condition que si x est égal à 0, y soit égal à au moins 2 et à la condition supplémentaire que y soit plus grand que x.
Selon un mode de mise en oeuvre, u vaut de 1 à 40 et t vaut de 0 à 30 à la condition que si t est égal à 0, u soit égal à au moins 2 et à la condition supplémentaire que u soit plus grand que t.
L'acide dicarboxylique peut être aliphatique et contenir de 2 à 36 atomes de carbone. Les acides dicarboxyliques aromatiques contiennent avantageusement de 8 à 36 atomes de carbone. Les acides dicarboxyliques aliphatiques préférés contiennent de 3 à 8 atomes de carbone. Les exemples d'acides dicarboxyliques aliphatiques appropriés comprennent l'acide adipique, l'acide sébacique, l'acide malonique, l'acide succinique et l'acide maléique.
Les acides dicarboxyliques aromatiques préférés contiennent de 8 à 12 atomes de carbone. Un exemple d'acide dicarboxylique aromatique approprié est l'acide phtalique. L'acide 1,2-phtalique, qui possède le point de fusion le plus bas des isomères de l'acide phtalique, est préféré.
De préférence, x et y peuvent chacun être égaux à 15, le total de x et y n'excédant pas 25. De préférence, u et t peuvent chacun être égaux à 15, le total de u et t n'excédant pas 25.
Dans un mode de réalisation, y ou u est supérieur ou égal à 1, et x ou t est supérieur ou égal à 0. Le nombre de motifs éthoxy peut être plus grand que le nombre de motifs propoxy.
Les esters alcoxylés peuvent être préparés selon l'enseignement du document WO 00/19972 dont le contenu est incorporé dans la présente demande par référence.
Les groupes aliphatiques saturés non substitués sont préférés pour les diesters d'acides dicarboxyliques de la présente invention, et de tels groupes gras contenant de 14 à 18 atomes de carbone sont davantage préférés. Encore plus préférés sont les groupes contenant de 14 à 16 atomes de carbone. Comme cela est mentionné précédemment, le groupe gras myristyle contenant 14 atomes de carbone est particulièrement préféré.
Dans un mode de réalisation préféré, lorsque R4 et R5 est un groupe myristyle, y et u est de préférence égal à zéro et chaque x et t est de préférence un entier choisi indépendamment parmi 2 à 40 inclus. Un exemple particulièrement préféré est le produit commercialisé sous la référence Cromollient DP3A dans lequel, dans la formule précédente, $R_4$ et $R_5$ sont un groupe myristyle, -OOC-B-COO- est un adipate, y=u est égal à 0 et x=t est égal à 3.
L'ester alcoxylé est avantageusement présent dans la composition à raison de 1 à 99 % en particulier, notamment de 2 à 60 % en poids, en particulier de 5 à 40 % et plus particulièrement de 10 à 35 % en poids par rapport au poids total de la composition.

**Polymère filmogène**

Par polymère "filmogène", on peut entendre un polymère apte à former à lui seul ou en présence d'un agent auxiliaire de filmification, un film continu sur un support, notamment sur les matières kératiniques, et de préférence un film cohésif et mieux encore, un film dont la cohésion et les propriétés mécaniques sont telles que ledit film peut être isolé dudit support.
Selon un mode de mise en oeuvre, le polymère filmogène n'est pas cristallin. Par exemple, le polymère est insoluble dans la ou les huiles de la compositions à sa température de ramollissement, à l'inverse d'une cire même d'origine polymérique qui est elle soluble la ou les huiles de la compositions à sa température de fusion.
Parmi les autres polymères filmogènes utilisables dans la composition de la présente invention, on peut citer les polymères synthétiques, de type radicalaire ou de type polycondensat, les polymères d'origine naturelle et leurs mélanges.
Comme polymère filmogène, on peut citer en particulier les polymères acryliques, les polyuréthanes, les polyesters, les polyamides, les polyurées, les polymères cellulosiques comme la nitrocellulose, les polymères siliconés, les polyamides siliconés.
Le polymère filmogène peut être utilisé en une quantité allant de 0,01 % à 50% par rapport au poids total de la composition, tel que par exemple de 1% à 30%, 5 à 25% le cas échéant.
Dans un mode de réalisation, le polymère filmogène est un polymère organique choisi parmi le groupe comprenant :

- les polymères filmogènes liposolubles,
- les polymères filmogènes lipodispersibles, en particulier les polymères sous la forme de dispersions non aqueuses de particules de polymères, de préférence des dispersions dans des huiles siliconées ou hydrocarbonées,
- les dispersions aqueuses de particules de polymères filmogènes, souvent appelées « latex » ; dans ce cas, la composition doit comprendre une phase aqueuse,
- les polymères filmogènes hydrosolubles ; dans ce cas, la composition doit comprendre une phase aqueuse.

**Dispersion de particules d'un polymère éthylénique greffé dans une phase grasse liquide.**

La composition selon l'invention peut comprendre, à titre d'agent filmogène, une dispersion de particules d'un polymère éthylénique greffé dans une phase grasse liquide.
Par polymère "éthylénique", on entend un polymère obtenu par polymérisation de monomères comprenant une insaturation éthylénique.
La dispersion de polymère éthylénique greffé est notamment exempte de polymère stabilisant distinct dudit polymère greffé, tels que ceux décrits dans EP749747 et décrits plus loin, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules.
Par polymère "greffé", on entend un polymère ayant un squelette comprenant au moins une chaîne latérale pendante ou située en bout de chaîne, et de préférence pendante.
Avantageusement, le polymère éthylénique greffé comprend un squelette éthylénique insoluble dans ladite phase grasse liquide, et des chaînes latérales liées de manière covalente audit squelette et solubles dans la phase grasse liquide.
Le polymère éthylénique greffé est notamment un polymère non réticulé. En particulier, le polymère est obtenu par polymérisation de monomères comprenant un seul groupement polymérisable.
Selon un mode de réalisation de l'invention, le polymère éthylénique greffé est un polymère acrylique greffé.
Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation du polymère éthylénique greffé.
Le milieu organique liquide de dispersion, correspondant au milieu dans lequel est fourni le polymère greffé, peut être identique au milieu de polymérisation.
Toutefois, le milieu de polymérisation peut être substitué en tout ou partie par un autre milieu organique liquide.

Cet autre milieu organique liquide peut être ajouté, après polymérisation, au milieu de polymérisation. Ce dernier est ensuite évaporé en tout ou partie.
La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.
Le milieu liquide organique de dispersion est présent dans la phase grasse liquide de la composition selon l'invention du fait de l'introduction dans la composition de la dispersion de polymère greffé obtenue.
La phase grasse liquide comprend, de préférence majoritairement un ou plusieurs composés organiques liquides (ou huiles) tels que définis ci-après.
En particulier, la phase grasse liquide est une phase organique liquide non aqueuse et non miscible à l'eau à la température ambiante (25 °C).
On entend par "composé organique liquide" un composé non aqueux qui est à l'état liquide à la température ambiante (25 °C) et qui s'écoule donc de son propre poids.
On entend par "composé siliconé" un composé contenant au moins un atome de silicium.
La composition selon l'invention contient avantageusement une huile volatile telle que décrite ci-après.
L'huile volatile peut être siliconée ou non siliconée. Elle peut être notamment choisie parmi l'octaméthylcyclotétrasiloxane, le décaméthylcyclopentasiloxane, le dodécaméthylcyclohexasiloxane, l'heptaméthyl-hexyltrisiloxane, l'heptaméthyloctyltrisiloxane, l'octaméthyltrisiloxane, le décaméthyltétrasiloxane, l'isododécane, l'isodécane, l'isohexadécane, et leurs mélanges.
L'huile volatile est avantageusement présente en une teneur allant de 1 % à 70 % en poids, par rapport au poids total de la composition, de préférence allant de 5 % à 50 % en poids, et préférentiellement allant de 10 % à 35 % en poids.
La phase grasse liquide peut contenir une huile non volatile telle que décrite ci-après. L'huile non volatile est avantageusement présente en une teneur allant de 1 % à 80 % en poids, par rapport au poids total de la composition, de préférence allant de 5 % à 60 % en poids, et préférentiellement allant de 10 % à 50 % en poids.
Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global δ selon l'espace de solubilité de Hansen est défini dans l'article « Solubility parameter values » de Eric A.Grulke de l'ouvrage « Polymer Handbook », 3éme édition, Chapitre VII, p.519-559 par la relation :

$$\delta = (\delta_D{}^2 + \delta_P{}^2 + \delta_H{}^2)^{1/2}$$

dans laquelle

- $d_D$ caractérise les forces de dispersion de LONDON issues de la formation de dipôles induits lors des chocs moléculaires,
- $d_P$ caractérise les forces d'intéractions de DEBYE entre dipôles permanents, et
- $d_H$ caractérise les forces d'intéractions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).
Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras liquides, notamment des huiles, qui peuvent être choisis parmi les huiles naturelles ou synthétiques, carbonées, hydrocarbonées, fluorées, siliconées, éventuellement ramifiées, seules ou en mélange.
Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne

hydrocarbonée comportant de 3 à 20 atomes de carbone, tels que les palmitates, les adipates et les benzoates, notamment l'adipate de diisopropyle.
On peut également citer les alcanes linéaires, ramifiés et/ou cycliques éventuellement volatils et notamment des huiles de paraffine, de vaseline, ou le polyisobutylène hydrogéné, l'isododécane, ou encore les 'ISOPARS', les isoparaffines volatiles. On peut citer également les esters, les éthers, les cétones.
On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.
En particulier, on peut citer les huiles de silicone, éventuellement ramifiées, volatiles et/ou non volatiles.
Par huile volatile, on entend une huile susceptible de s'évaporer de la peau ou des lèvres en moins d'une heure, ayant notamment une pression de vapeur, à température ambiante et pression atmosphérique allant de $10^{-3}$ à 300 mm de Hg (0,13 Pa à 40 000 Pa).
Comme huile siliconée volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. En particulier, on peut citer l'octaméthylcyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.
Comme huile siliconée non volatile, on peut citer les polydialkylsiloxanes non volatils, tels que les polydiméthylsiloxanes (PDMS) non volatils; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényl triméthicones, les phényl diméthicones, les phényl triméthylsiloxy diphénylsiloxanes, les diphényl diméthicones, les diphényl méthyldiphényl trisiloxanes, les polyméthylphénylsiloxanes; les polysiloxanes modifiés par des acides gras (notamment en $C_8$-$C_{20}$), des alcools gras (notamment en $C_8$-$C_{20}$) ou des polyoxyalkylènes (notamment polyoxyéthyléne et/ou polyoxypropylène); les polysiloxanes aminées ; les polysiloxanes à groupement hydroxyles; les polysiloxanes fluorés comportant un groupement fluoré pendant ou en bout de chaîne siliconée ayant de 1 à 12 atomes de carbone dont tout ou partie des hydrogène sont substitués par des atomes de fluor ; et leurs mélanges.

On peut citer, en particulier, comme composés organiques liquides non siliconés ayant un paramètre de solubilité global selon l'espace de solubilité de Hansen inférieur ou égal à 18 $(Mpa)^{1/2}$ :

- les esters linéaires, ramifiés ou cycliques, ayant au moins 6 atomes de carbone, notamment de 6 à 30 atomes de carbone;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, l'hexyldécanol, le décanol, l'octyldodécanol et l'alcool linoléique.

Selon un premier mode de réalisation de l'invention, la phase grasse liquide peut être une phase grasse liquide non siliconée.
On entend par "phase grasse liquide non siliconée" une phase grasse comprenant un ou plusieurs composés organiques liquides ou huiles non siliconé(e)s, tels que ceux cités précédemment, lesdits composés non siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides non siliconés peuvent être notamment choisis parmi :

- les composés organiques liquides non siliconés ayant un paramètre de solubilité global selon l'espace de solubilité de Hansen inférieur ou égal à 18 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Ladite phase grasse liquide non siliconée peut donc éventuellement comprendre des composés organiques liquide ou huiles siliconé(e)s, tels que ceux cités précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide non siliconée ne contient pas de composés organiques liquides ou huiles siliconé(e)s.

Lorsque la phase grasse liquide est une phase grasse liquide non siliconée, les macromonères présents dans le polymère greffé sont avantageusement des macromonomères carbonés tels que décrits ci-après.

En particulier, lorsque la phase grasse liquide est une phase grasse liquide non siliconée, le polymère greffé présent dans la composition est avantageusement un polymère greffé non siliconé.

Par polymère greffé non siliconé, on entend un polymère greffé contenant majoritairement un macromonomère carboné et contenant éventuellement au plus 7 % en poids, de préférence au plus 5 % en poids, voire est exempt, de macromonomère siliconé.

Selon un deuxième mode de réalisation de l'invention, la phase grasse liquide peut être une phase grasse liquide siliconée.

On entend par "phase grasse liquide siliconée" une phase grasse comprenant un ou plusieurs composés organiques liquides siliconés ou huiles siliconées tels que ceux décrits précédemment, lesdits composés siliconés étant présents majoritairement dans la phase grasse liquide, c'est-à-dire à au moins 50 % en poids, notamment de 50 à 100 % en poids, de préférence de 60 % à 100 % en poids (par exemple de 60 à 99 % en poids), ou encore de 65 % à 100 % en poids (par exemple de 65 à 95 % en poids), par rapport au poids total de la phase grasse liquide.

Les composés organiques liquides siliconés peuvent être notamment choisis parmi les composés organiques liquides siliconés, ayant un paramètre de solubilité global selon l'espace de solubilité de Hansen inférieur ou égal à 18 $(MPa)^{1/2}$.

Ladite phase grasse liquide siliconée peut donc éventuellement comprendre des composés organiques liquides ou huiles non siliconé(e)s, tels que décrits précédemment, qui peuvent être présents en une quantité inférieure à 50 % en poids, notamment allant de 0,1 à 40 % en poids, voire allant de 1 à 35 % en poids, ou encore allant de 5 à 30 % en poids, par rapport au poids total de la phase grasse liquide.

Selon un mode particulier de réalisation de l'invention, la phase grasse liquide siliconée ne contient pas de composés organiques liquides non siliconés.

Lorsque la phase grasse liquide est une phase grasse liquide siliconée, les macromonères présents dans le polymère greffé sont avantageusement des macromonomère siliconés tels que décrits ci-après.

En particulier, lorsque la phase grasse liquide est une phase grasse liquide siliconée, le polymère greffé présent dans la composition est avantageusement un polymère greffé siliconé.

Par polymère greffé siliconé, on entend un polymère greffé contenant majoritairement un macromonomère siliconé et contenant éventuellement au plus 7 % en poids, de préférence au plus 5 % en poids, voire est exempt, de macromonomère carboné.

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère éthylénique greffé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé " un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé "un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

De préférence, les monomères acryliques sont choisis parmi les monomères dont l'homopolymère est insoluble

dans le milieu de dispersion considéré, c'est-à-dire que l'homopolymère est sous forme solide (ou non dissous) à une concentration supérieure ou égale à 5% en poids à température ambiante (20˚C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

De préférence, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersionconsidéré alors que les chaînes latérales (ou greffons) sont solubles dans ledit milieu de dispersion.

Par "monomère acryliques", on entend dans la présente demande des monomères choisis parmi l'acide (méth) acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule :

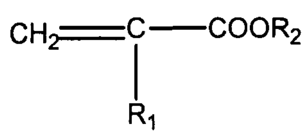

dans laquelle :

- $R_1$ désigne un atome d'hydrogène ou un groupe méthyle ;
- $R_2$ représente un groupe choisi parmi :
  - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE , trifluoroéthyle, 2-hydroxyéthyle, 2-hydroxypropyle, diméthylaminoéthyle, diéthylaminoéthyle, diméthytaminopropyle.

- (ii) les (méth)acrylamides de formule :

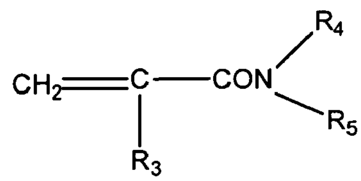

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle.

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.
De préférence, les monomères acryliques sont choisis parmi l'acrylate de méthyle, l'acrylate de méthoxyéthyle, le méthacrylate de méthyle, le méthacrylate de 2-hydroxyéthyle, l'acide acrylique, le méthacrylate de diméthylaminoéthyle, et leurs mélanges.
Parmi les monomères additionnels vinyliques non acryliques, on peut citer :

- les esters vinylique de formule : $R_6$-COO-CH=$CH_2$
dans laquelle $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

  - les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;
  - les monomères vinyliques non acryliques comprenant au moins une fonction amine tertiaire, tels que la 2-vinylpyridine, la 4-vinylpyridine ;
  - et leurs mélanges.

Avantageusement, les monomères acryliques présents dans le polymère greffés comprennent au moins l'acide (méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.
Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.
On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un

ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.

Il est entendu que ces monomères acryliques non polymérisés peuvent être solubles dans le milieu de dispersion considéré, mais le polymère formé avec ces monomères est insoluble dans le milieu de dispersion.

Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide (meth)acrylique, l'acide méthacrylique et les (méth)acrylates d'alkyle de formule (I) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'iso-propyle, et leurs mélanges.
On préfère tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (I) et leurs sels :

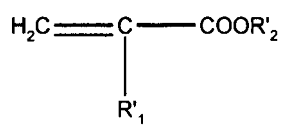

(I)

dans laquelle :

- R'$_1$ désigne un atome d'hydrogène ou un groupe méthyle ;
- R'$_2$ représente
- un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi
- OH, les atomes d'halogène (F, Cl, Br, I) et -NR'R" avec R' et R" identiques ou différents choisis parmi les alkyles linéaires ou ramifiés en $C_1$-$C_3$ ;

  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;
  - et leurs mélanges.

A titre d'exemples de R'$_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.
On préfère tout particulièrement l'acide acrylique, l'acide méthylacrylique.

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut

être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600. De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8-C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth) acrylate.

De tels macromonomères sont notamment décrits dans les brevets EP895467 et EP96459 et dans l'article Gillman K.F., Polymer Letters, Vol 5, page 477-481 (1967).

On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;

De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination Kraton Liquid L-1253 par Kraton Polymers.

Comme macromonomères siliconés, on peut en particulier citer les polydiméthylsiloxanes à groupement terminal mono (méth)acrylate, et notamment ceux de formule (II) suivante :

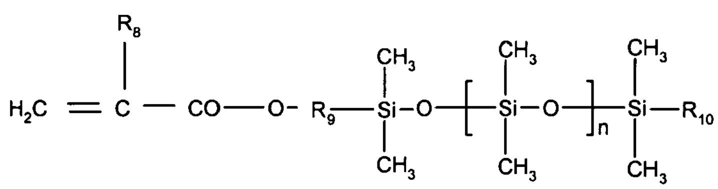
(II)

dans laquelle $R_8$ désigne un atome d'hydrogène ou un groupement méthyle $R_9$ désigne un groupe hydrocarboné divalent ayant de 1 à 10 atomes de carbone et contient éventuellement une ou deux liaisons éther -O- ; R10 désigne un groupe alkyl ayant de 1 à 10 atomes de carbone, notamment de 2 à 8 atomes de carbone ; n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société United Chemical Technologies Inc. (UCT) ou sous la dénomination MCR-M17 par la société Gelest Inc.

De préférence, le macromonomère polymérisé (constituant les chaînes latérales du polymère greffé) représente

de 0,1 à 15 % en poids du poids total du polymère, préférentiellement de 0,2 à 10 % en poids, et plus préférentiellement de 0,3 à 8 % en poids.

Comme polymère éthylénique greffé particulièrement préféré dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment Kraton L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment Kraton L-1253), en particulier dans l'isododécane ;
- des monomères acrylate de méthyle / méthacrylate de méthyle et du macromonomère polyéthylène/polybutylène à groupement terminal méthacrylate (notamment Kraton L-1253), en particulier dans l'isododécane ;
- des monomères acrylate de méthyle / acide acrylique et du macromonomère polyéthylène/polybutylène à groupement terminal méthacrylate (notamment Kraton L-1253), en particulier dans l'isododécane ;
- des monomères acrylate de méthyle / méthacrylate de diméthylaminoéthyle et du macromonomère polyéthylène/ polybutylène à groupement terminal méthacrylate (notamment Kraton L-1253), en particulier dans l'isododécane ;
- des monomères acrylate de méthyle / méthacrylate de 2-hydroxyéthyle et du macromonomère polyéthylène/polybutylène à groupement terminal méthacrylate (notamment Kraton L-1253), en particulier dans l'isododécane.

Comme polymère acrylique greffé particulièrement préféré dispersé dans une phase grasse liquide siliconée, on peut utiliser ceux obtenus par polymérisation :

- de l'acrylate de méthyle et du macromonomère monométhacryloylxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

De préférence, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.

En particulier, les polymères éthyléniques greffés de la dispersion peuvent former des particules nanométriques, de taille moyenne allant de 10 à 400 nm, de préférence de 20 à 200 nm.

Du fait de cette taille très faible, les particules de polymère greffé en dispersion sont particulièrement stables et donc peu susceptibles de former des agglomérats.

La dispersion de polymère greffé peut donc être une dispersion stable et ne forme pas de sédiments, lorsqu'elle est placée pendant une durée prolongée (par exemple 24 heures) à température ambiante (25 °C).

De préférence, la dispersion de particules de polymère greffé présente un taux de matière sèche (ou extrait sec) en polymère pouvant aller de 40 % à 70 % en poids de matière sèche, notamment allant de 45 % à 65 % en poids.

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.

Comme indiqué précédemment, le milieu organique liquide de dispersion peut être identique ou différent du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu organique de polymérisation, une partie des

monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.
Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.
Le polymère greffé peut être présent dans la composition selon l'invention en une teneur en matière sèche (ou matière active) allant de 1 à 70% en poids par rapport au poids total de la composition, mieux de 5 à 60% en poids, de préférence allant de 6 à 45% et mieux allant de 8 à 40% en poids.

**Polymère éthylénique séquencé linéaire**

La composition selon l'invention peut contenir, à titre d'agent filmogène un polymère éthylénique séquencé linéaire, appelé par la suite "polymère séquencé", de structure particulière telle que décrite ci-après.
Par polymère "séquencé", on entend un polymère comprenant au moins 2 séquences distinctes, de préférence au moins 3 séquences distinctes.
Le polymère est un polymère à structure linéaire. Par opposition, un polymère de structure non linéaire est, par exemple, un polymère à structure ramifiée, en étoile, greffée, ou autre.
Avantageusement, le polymère séquencé est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène.
De préférence, le polymère séquencé comprend au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Par "au moins" une séquence, on entend une ou plusieurs séquences.
La séquence intermédiaire est une séquence comprenant au moins un monomère constitutif de la première séquence et au moins un monomère constitutif de la deuxième séquence du polymère permet de "compatibiliser" ces séquences.
On précise que dans ce qui précède et ce qui suit les termes "première" et "deuxième" séquences ne conditionnent nullement l'ordre desdites séquences (ou blocs) dans la structure du polymère séquencé.
Avantageusement, les première et deuxième séquences et du polymère séquencé sont incompatibles l'une avec l'autre.
Par "séquences incompatibles l'une avec l'autre", on entend que le mélange formé du polymère correspondant à la première séquence et du polymère correspondant à la deuxième séquence, n'est pas miscible dans le liquide organique majoritaire en poids du la phase grasse liquide, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

i) lesdits polymères sont présents dans le mélange en une teneur telle que le rapport pondéral respectif va de 10/90 à 90/10, et que
ii) chacun des polymères correspondant au première et seconde séquences a une masse moléculaire moyenne (en poids ou en nombre) égale à celle du polymère séquencé +/- 15%.

Dans le cas où la phase grasse liquide comprend un mélange de liquide organiques, et dans l'hypothèse de deux ou plusieurs liquides organiques présents en proportions massiques identiques, ledit mélange de polymères est non miscible dans au moins l'un d'entre eux.
Bien entendu, dans le cas où la phase grasse liquide comprend un unique liquide organique, ce dernier est le liquide organique majoritaire.
De façon préférentielle, le polymère séquencé ne comprend pas d'atomes de silicium dans son squelette. Par "squelette", on entend la chaîne principale du polymère, par opposition aux chaînes latérales pendantes.
De préférence, le polymère séquencé n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans

modification de pH, à une teneur en matière active d'au moins 1% en poids, à température ambiante (25°C).
De préférence, le polymère séquencé n'est pas un élastomère.
Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.
De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.
Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.
On obtient alors un film d'environ 100 μm d'épaisseur dans lequel sont découpées des éprouvettes rectangulaires (par exemple à l'emporte-pièce) d'une largeur de 15 mm et d'une longueur de 80 mm.
On impose à cet échantillon une sollicitation de traction à l'aide d'un appareil commercialisé sous la référence Zwick, dans les mêmes conditions de température et d'humidité que pour le séchage.
Les éprouvettes sont étirées à une vitesse de 50 mm/min et la distance entre les mors est de 50 mm, ce qui correspond à la longueur initiale ($l_0$) de l'éprouvette.

On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.
La recouvrance retardée en % ($R_{2h}$) est donnée par la formule ci-après:

$$R_{2h}= (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.
Avantageusement, le polymère séquencé a un indice de polydispersité 1 supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.
L'indice de polydispersité 1 du polymère séquencé est égal au rapport de la masse moyenne en poids Mw sur la masse moyenne en nombre Mn.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).
La masse moyenne en poids (Mw) du polymère séquencé est de préférence inférieure ou égale à 300 000, elle va par exemple de 35 000 à 200 000, et mieux de 45 000 à 150 000.
La masse moyenne en nombre (Mn) du polymère séquencé est de préférence inférieure ou égale à 70 000, elle va par exemple de 10 000 à 60 000, et mieux de 12 000 à 50 000.
Chaque séquence ou bloc du polymère séquencé est issue d'un type de monomère ou de plusieurs types de monomères différents.
Cela signifie que chaque séquence peut être constituée d'un homopolymère ou d'un copolymère ; ce copolymère constituant la séquence pouvant être à son tour statistique ou alterné.
Avantageusement, la séquence intermédiaire comprenant au moins un monomère constitutif de la première séquence

et au moins un monomère constitutif de la deuxième séquence du polymère séuqnecé est un polymère statistique.
De préférence, la séquence intermédiaire est issue essentiellement de monomères constitutifs de la première séquence et de la deuxième séquence.
Par "essentiellement", on entend au moins à 85%, de préférence au moins à 90%, mieux à 95% et encore mieux à 100%.
Avantageusement, la séquence intermédiaire a une température de transition vitreuse Tg comprise entre les températures de transition vitreuse des première et deuxième séquences.
Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg= \sum_i (\varpi_i / Tg_i) ,$$

$\varpi_i$ étant la fraction massique du monomère i dans la séquence considerée et $Tg_i$ étant la température de transition vitreuse de l'homopolymère du monomère i.
Sauf indication contraire, les Tg indiquées pour les première et deuxième séquences dans la présente demande sont des Tg théoriques.
L'écart entre les températures de transition vitreuse des première et deuxième séquences est généralement supérieur à 10°C, de préférence supérieur à 20°C, et mieux supérieur à 30°C.
En particulier, la première séquence du polymère séquencé peut être choisie parmi :

a) une séquence ayant une Tg supérieure ou égale à 40°C,
b) une séquence ayant une Tg inférieure ou égale à 20°C,
c) une séquence ayant une Tg comprise entre 20 et 40°C,

et la deuxième séquence choisie dans une catégorie a), b) ou c) différente de la première séquence.
On entend désigner dans la présente invention, par l'expression :

« compris entre ... et ... », un intervalle de valeurs dont les bornes mentionnées sont exclues, et
« de ... à ... » et « allant de ... à ... », un intervalle de valeurs dont les bornes sont inclues.

<u>a) Séquence avant une Tg supérieure ou égale à 40°C</u>

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.
La séquence ayant une Tg supérieure ou égale à 40°C peut être un homopolymère ou un copolymère.
Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).
Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20 à 40°C et/ou les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin,

Les monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule $CH_2 = CH\text{-}COOR_2$
  dans laquelle $R_2$ représente un groupe cycloalkyle en $C_4$ à $C_{12}$ tel que l'acrylate d'isobornyle ou un groupe tertio butyle,

- les (méth)acrylamides de formule :

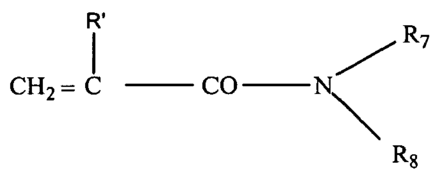

  où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
  et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

Des monomères principaux particulièrement préférés sont le méthacrylate de méthyle, le (méth)acrylate d'isobutyle, le (méth)acrylate d'isobornyle et leurs mélanges.

b) Séquence avant une Tg inférieure ou égale à 20°C

La séquence ayant une Tg inférieure ou égale à 20°C a par exemple une Tg allant de -100 à 20°C, de préférence inférieure ou égale à 15°C, notamment allant de -80°C à 15°C et mieux inférieure ou égale à 10°C, par exemple allant de - 50°C à 0°C.

La séquence ayant une Tg inférieure ou égale à 20°C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle,

dans lequel se trouve(nt) éventuellement intercalé(s) un ou plusieurs hétéroatomes choisis parmi O, N, S,

- les méthacrylates de formule $CH_2 = C(CH_3)-COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5$-CO-O-CH = $CH_2$
  où $R_5$ représente un groupe alkyle en $C_4$ à $C_{12}$ linéaire ou ramifié ;

- les éthers d'alcool vinylique et d'alcool en $C_4$ à $C_{12}$,

- les N-alkyl en $C_4$ à $C_{12}$ acrylamides, tels que le N-octylacrylamide,

- et leurs mélanges.

Les monomères principaux particulièrement préférés pour la séquence ayant une Tg inférieure ou égale à 20˚C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

c) Séquence avant une Tg comprise entre 20 et 40˚C

La séquence qui a une Tg comprise entre 20 et 40˚C peut être un homopolymère ou un copolymère.
Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40˚C. Cette première séquence peut être un homopolymère, constitué par un seul type de monomère (dont la Tg de l'homopolymère correspondant va de 20˚C à 40˚C).
Les monomères dont l'homopolymère a une température de transition vitreuse comprise entre 20 et 40˚C sont, de préférence, choisis parmi le méthacrylate de n-butyle, l'acrylate de cyclodécyle, l'acrylate de néopentyle, l'isodécylacrylamide et leurs mélanges.
Dans le cas où la séquence ayant une Tg comprise entre 20 et 40˚C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40˚C.
Avantageusement, la séquence ayant une Tg comprise entre 20 et 40˚C est un copolymère issue en totalité ou en partie :

- de monomères principaux dont l'homopolymère correspondant a une Tg supérieure ou égale à 40˚C, par exemple une Tg allant de 40˚C à 150˚C, de préférence supérieure ou égale à 50˚C, allant par exemple de 50 à 120˚C, et mieux supérieure ou égale à 60˚C, allant par exemple de 60˚C à 120˚C, tels que décrits plus haut, et/ou
- de monomères principaux dont l'homopolymère correspondant a une Tg inférieure ou égale à 20˚C, par exemple une Tg allant de -100 à 20˚C, de préférence inférieure ou égale à 15˚C, notamment allant de -80˚C à 15˚C et mieux inférieure ou égale à 10˚C, par exemple allant de -50˚C à 0˚C, tels que décrits plus haut,
  lesdits monomères étant choisis de telle sorte que la Tg du copolymère formant la première séquence est comprise entre 20 et 40˚C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
De préférence, la proportion de la deuxième séquence ayant une Tg inférieure ou égale à 20˚C va de 10 à 85% en poids du polymère, mieux de 20 à 70% et encore mieux de 20 à 50%.
Chacune des séquences peut néanmoins contenir en proportion minoritaire au moins un monomère constitutif de l'autre séquence.
Ainsi la première séquence peut contenir au moins un monomère constitutif de la deuxième séquence et inversement.
Chacune des première et/ou deuxième séquence du polymère séquencé peu(ven)t comprendre, outre les monomères indiqués ci-dessus, un ou plusieurs autres monomères appelés monomères additionnels, différents des monomères principaux cités précédemment.
La nature et la quantité de ce ou ces monomères additionnels sont choisies de manière à ce que la séquence dans laquelle ils se trouvent ait la température de transition vitreuse désirée.
Ce monomère additionnel est par exemple choisi parmi :

les monomères hydrophiles tels que :

- les monomères à insaturation(s) éthylénique(s) comprenant au moins une fonction acide carboxylique ou sulfonique comme par exemple :

l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
- les méthacrylates de formule $CH_2 = C(CH_3)-COOR_6$
dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, 1, F), tel que le méthacrylate de trifluoroéthyle,
- les méthacrylates de formule $CH_2 = C(CH_3)-COOR_9$,
$R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$,
$R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène

b) les monomères à insaturation éthylénique comprenant un ou plusieurs atomes de silicium tels que le méthacryloxypropyl triméthoxy silane, le méthacryloxypropyl tris (triméthylsiloxy ) silane,

- et leurs mélanges.

Des monomères additionnels particulièrement préférés sont l'acide acrylique, l'acide méthacrylique, le méthacrylate de trifluoroéthyle et leurs mélanges.
Selon un mode préféré de réalisation, le polymère séquencé est un polymère non siliconé, c'est à dire un polymère exempt d'atome de silicium.
Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.
De préférence, chacune des première et deuxième séquences comprend au moins un monomère choisi parmi les esters d'acide (méth)acrylique, et éventuellement au moins un monomère choisi parmi l'acide (méth)acrylique, et leurs mélanges.
Avantageusement, chacune des première et deuxième séquences du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges
Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les

alcanes aliphatiques tels que l'isododécane et leurs mélanges. De préférence, le solvant de polymérisation est un mélange acétate de butyle et isopropanol ou l'isododécane.

Selon un premier mode de réalisation, le polymère séquencé comprend une première séquence ayant une Tg supérieure ou égale à 40°C, telle que décrite plus haut au a) et une deuxième séquence ayant une Tg inférieure ou égale à 20°C, telle que décrite plus haut au b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomère décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Avantageusement, le polymère séquencé peut comprendre :

- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon un second mode de réalisation, le polymère séquencé comprend une première séquence ayant une température de transition vitreuse (Tg) comprise entre 20 et 40°C, conforme aux séquences décrites au c) et une deuxième séquence ayant une température de transition vitreuse inférieure ou égale à 20°C, telle que décrite plus haut au b) ou une température de transition vitreuse supérieure ou égale à 40°C, telle que décrite au a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

.Avantageusement, la deuxième séquence ayant une Tg inférieure ou égale à 20°C ou ayant une Tg supérieure ou égale à 40°C est un homopolymère.

Selon une première variante, le polymère séquencé comprend :

- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une deuxième variante, le polymère séquencé peut comprendre :

- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon une troisième variante, le polymère séquencé peut comprendre :

- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Le polymère filmogène peut être d'un type chimique quelconque et peut être notamment choisi parmi:

a) les homopolymères et les copolymères liposolubles et amorphes des oléfines, des cyclooléfines, du butadiène, de l'isoprène, du styrène, des éthers, des esters ou amides vinyliques, des esters ou amides de l'acide (méth) acrylique contenant un groupement alkyle en $C_{4-50}$ linéaire, ramifié ou cyclique, et préférablement amorphes. Les homopolymères et les copolymères liposolubles préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML, et les copolymères de vinylpyrrolidone, tels que les copolymères d'un alkène en $C_2$ à $C_{30}$, tel qu'en $C_3$ à $C_{22}$, et des associations de ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/triacontène ou de VP/acide acrylique/méthacrylate de lauryle.
Comme copolymères liposolubles particuliers, on peut citer :

i) les polymères de silicone-acrylique greffés ayant un squelette siliconé, des greffons acryliques ou ayant un squelette acrylique, les greffons de silicone tels que le produit commercialisé sous la dénomination SA 70.5 par 3M et décrit dans les brevets US 5 725 882, US 5 209 924, US 4 972 037, US 4 981 903, US 4 981 902, US 5 468 477, et dans les brevets US 5 219 560 et EP 0 388 582.

ii) les polymères liposolubles portant des groupements fluorés appartenant à l'une des classes décrites dans le texte ci-dessus, en particulier Fomblin ceux décrits dans le brevet US 5 948 393, les copolymères de (méth) acrylate d'alkyle/(méth)acrylate de perfluoroalkyle décrits dans les brevets EP 0 815 836 et US 5 849 318.

iii) les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.
Dans un mode de réalisation, l'agent filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « Luvitol HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés.
On peut citer par exemple le le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel 5970 et le Versagel 5960 de chez Penreco (mélange de polymère en étoile et de polymère tribloc dans l'isododécane).
Des copolymères de styrène-méthacrylate peuvent également être utilisés tels que les polymère commercialisés

sous les référenceq OS 129880, OS 129881 et OS 84383 de chez Lubrizol (copolymère de styrène-méthacrylate).

Dans un mode de réalisation, l'agent filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly (méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme exemples de polymères liposolubles pouvant être utilisés dans l'invention, on peut citer les polyalkylènes, les copolymères d'alcènes en $C_2$-$C_{20}$, en particulier le polybutène.

b) les polycondensats amorphes et liposolubles, de préférence ne comprenant pas de groupements donneurs d'interactions hydrogène, en particulier les polyesters aliphatiques ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, tel que défini dans la demande de brevet FR 0 113 920.

c) les polysaccharides amorphes et liposolubles comprenant des chaînes latérales alkyl (éther ou ester), en particulier les alkylcelluloses ayant un radical alkyle en $C_1$ à $C_8$ saturé ou insaturé, linéaire ou ramifié, tel que l'éthylcellulose et la propylcellulose.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résine toluène sulfonamide formaldéhyde "Ketjentflex MS80" de la société AKZO ou "Santolite MHP", "Santolite MS 80" ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

d) les polymères siliconés, comme les gommes de silicones et les résines de silicone.

La viscosité des gommes de silicone peut être comprise entre 1 000 et 10 000 000 cSt, de préférence entre 100 000 et 1 000 000 cSt, de préférence encore entre 300 000 et 700 000 cSt, mesurée selon la norme ASTM D-445. La gomme de silicone est de préférence choisie parmi les diméthiconols, les fluorosilicones, les diméthicones et leurs mélanges. Les diméthicones incluent les diméthicones décrites dans le brevet US 4 152 416. Elles sont par exemple commercialisées sous les références SE30, SE33, SE 54 et SE 76.
Comme gomme de silicone utilisable selon l'invention, on peut citer celle vendue sous la dénomination SE30 par la société Général Electric, celle vendue sous la dénomination AK 500000 par la société Waker, celle vendue sous la dénomination Silbione 70047 V par la société Rhodia, celle vendue sous la dénomination Q2-1401 ou Q2-1403 par la société Dow Corning, et celle vendue sous la dénomination 761 par la société Rhône-Poulenc.
Les résines de silicones peuvent être solubles ou gonflables dans les huiles de silicone. Ces résines sont des polymères de polyorganosiloxanes réticulés.
La nomenclature des résines de silicone est connue sous le nom de "MDTQ", la résine étant décrite en fonction des différentes unités monoméres siloxane qu'elle comprend, chacune des lettres "MDTQ" caractérisant un type d'unité.
La lettre M représente l'unité monofonctionelle de formule $(CH_3)_3SiO_{1/2}$, l'atome de silicium étant relié à un seul atome d'oxygène dans le polymère comprenant cette unité.
La lettre D signifie une unité difonctionelle $(CH_3)_2SiO_{2/2}$ dans laquelle l'atome de silicium est relié à deux atomes d'oxygène
La lettre T représente une unité trifonctionnelle de formule $(CH_3)SiO_{3/2}$.
Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.
Enfin, la lettre Q signifie une unité tétra fonctionnelle $SiO_{4/2}$ dans laquelle l'atome de silicium est lié à quatre atomes d'hydrogène eux mêmes liés au reste du polymère.
Divers résines de propriétés différentes peuvent être obtenues à partir de ces différentes unités, les propriétés des ces polymères variant en fonction du type de monomères (ou unités), du type et du nombre de radicaux substitués, de la longueur de la chaîne polymérique, du degré de ramification et de la taille des chaînes pendantes.
A titre d'exemple de ces résines silicones, on peut citer :

- les siloxysilicates qui peuvent être des triméthylsiloxysilicate de formule $[(CH_3)_3XSiXO]_xX(SiO_{4/2})_y$ (unités MQ) dans laquelle x et y sont des entiers allant de 50 à 80,
- les polysilesquioxanes de formule $(CH_3SiO_{3/2})_{.x}$ (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.

A titre d'exemples de résines polymethylsilsesquioxanes commercialement disponibles, on peut citer celles qui sont commercialisés :

- par la société Wacker sous la référence Resin MK tels que la Belsil PMS MK : polymère comprenant des unités répétitives $CH_3SiO_{3/2}$ (unités T), pouvant aussi comprendre jusqu'à 1% en poids d'unités $(CH_3)_2SiO_{2/2}$ (unités D) et présentant un poids moléculaire moyen d'environ 10000,
- par la société SHIN-ETSU sous les références KR-220L qui sont composé d'unités T de formule $CH_3SiO_{3/2}$ et ont des groupes terminaux Si-OH (silanol), sous la référence KR-242A qui comprennent 98% d'unités T et 2% d'unités diméthyle D et ont des groupes terminaux Si-Ohou encore sous la référence KR-251 comprenant 88% d'unités T et 12% d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

e) Les polyamides siliconés du type polyorganosiloxane tels que ceux décrits dans les documents US-A-5 874 069, US-A-5,919,441, US-A-6,051,216 et US-A-5,981,680.
Selon l'invention, ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.
Les polymères comportant deux groupes capables d'établir des interactions hydrogène dans la chaîne du polymère peuvent être des polymères comprenant au moins un motif répondant à la formule :

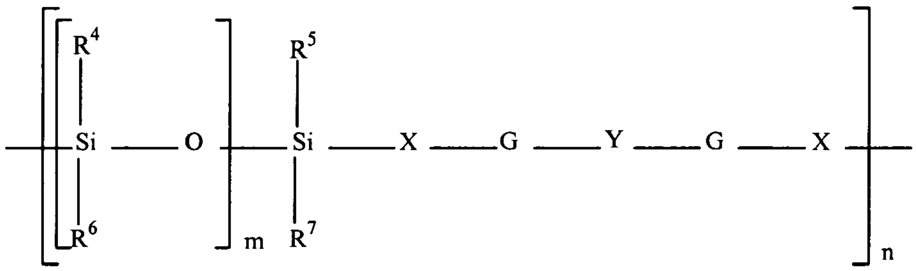

(II)

dans laquelle :

1) $R^4$, $R^5$, $R^6$ et $R^7$, identiques ou différents, représentent un groupe choisi parmi:

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4) Y représente un groupe répondant à la formule :

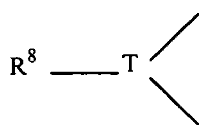

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

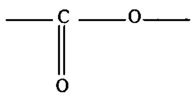 ; 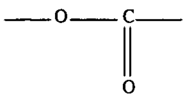 ; 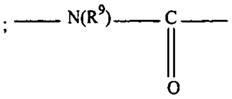 ;

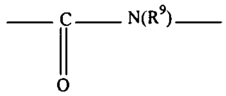 ; 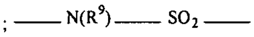 ; 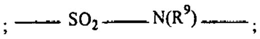 ;

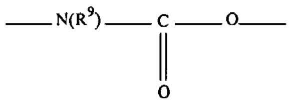 ; 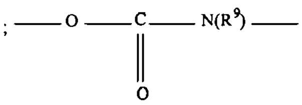 ; 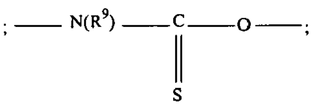 ;

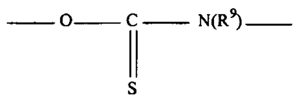 ; 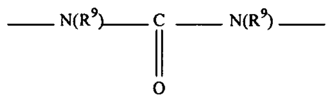 ,

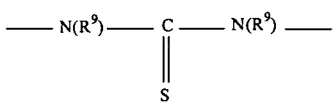 ,

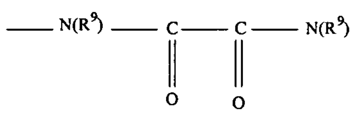 ; 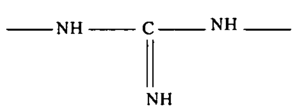 ; et

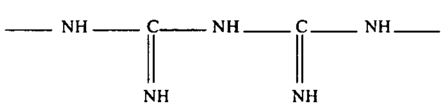

où $R^9$ représente un atome d'hydrogène ou un groupe alkyle, linéaire ou ramifié, en $C_1$ à $C_{20}$, à condition qu'au moins 50% des $R^9$ du polymère représente un atome d'hydrogène et qu'au moins deux des groupes G du polymère soient un autre groupe que :

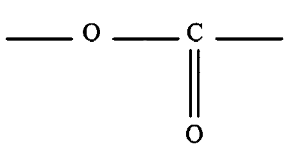 et 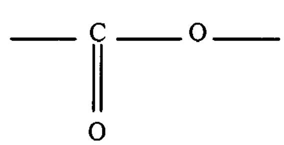 ;

6) n est un nombre entier allant de 2 à 500, de préférence de 2 à 200, et m est un nombre entier allant de 1 à 1000, de préférence de 1 à 700 et mieux encore de 6 à 200.

Selon l'invention, 80% des $R^4$, $R^5$, $R^6$ et $R^7$, du polymère sont choisis de préférence parmi les groupes méthyle,

éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule :

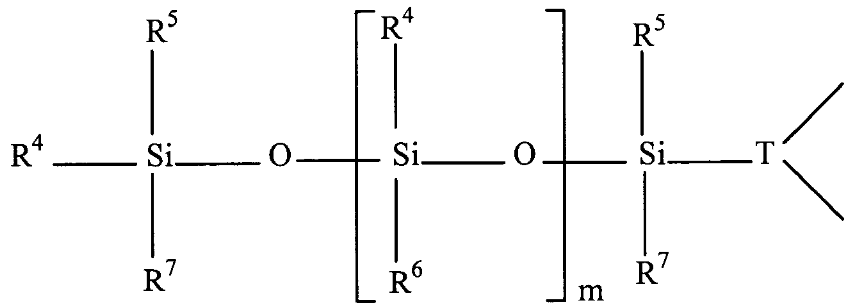

dans laquelle $R^4$, $R^5$, $R^6$, $R^7$, T et m sont tels que définis ci-dessus, et
h) les chaînes polyorganosiloxanes de formule :

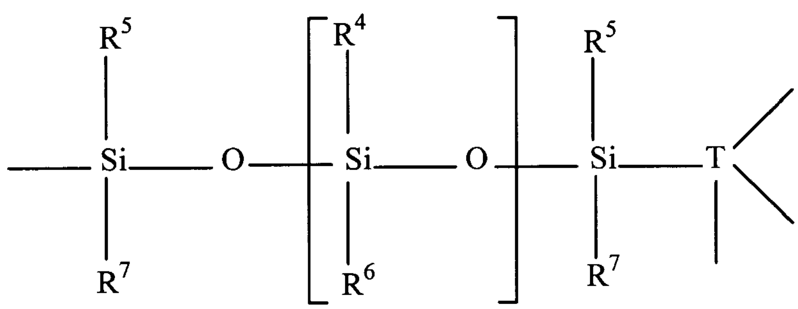

Les polyorganosiloxanes de la seconde famille peuvent être des polymères comprenant au moins un motif répondant à la formule (III) :

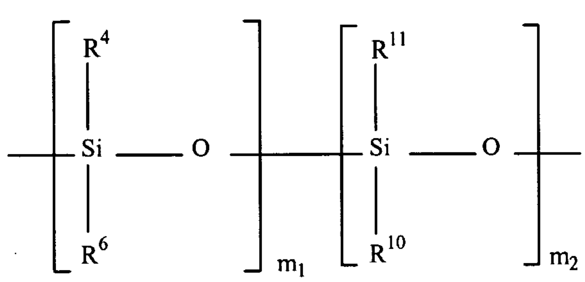 (III)

dans laquelle

- $R^4$ et $R^6$, identiques ou différents, sont tels que définis ci-dessus pour la formule (II),
- $R^{10}$ représente un groupe tel que défini ci-dessus pour $R^4$ et $R^6$, ou représente le groupe de formule -X-G-$R^{12}$ dans laquelle X et G sont tels que définis ci-dessus pour la formule (II) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
- $R^{11}$ représente le groupe de formule -X-G-$R^9$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère utilisé, peut être un homopolymère, c'est-à-dire un polymère comportant plusieurs motifs identiques, en particulier des motifs de formule (II) ou de formule (III).
Selon l'invention, on peut aussi utiliser un polymère constitué par un copolymère comportant plusieurs motifs de formule (II) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (III), dans lequel l'un au moins des $R^4$, $R^6$, $R^{10}$, $R^{11}$, $m_1$ et $m_2$ est différent dans l'un au moins des motifs.
On peut encore utiliser un copolymère comportant au moins un motif de formule (II) et au moins un motif de formule (III), les motifs de formule (II) et les motifs de formule (III) pouvant être identiques ou différents les uns des autres.
Selon une variante, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.
Ces copolymères peuvent être des copolymères blocs, des copolymères séquencés ou des copolymères greffés.

**Dispersion de particules de polymère dans une phase grasse liquide**

La composition selon l'invention comprend avantageusement au moins une dispersion stable de particules de polymère essentiellement sphériques d'un ou plusieurs polymères, dans une phase grasse liquide physiologiquement acceptable.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1$\mu$m.

De préférence, les particules de polymères en dispersion sont insolubles dans les alcools hydrosolubles tels que, par exemple, l'éthanol.

Les polymères en dispersion utilisables dans la composition de l'invention ont de préférence un poids moléculaire de l'ordre de 2000 à 10 000 000 g/mol, et une Tg de -100°C à 300°C et mieux de -50° à 100°C, de préférence de -10°C à 50°C.

Il est possible d'utiliser des polymères filmifiables, de préférence ayant une Tg basse, inférieure ou égale à la température de la peau et notamment inférieure ou égale à 40°C.

Parmi les polymères filmogènes, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de

benzyle ou de phényle.

Les esters de l'acide (méth)acrylique particulièrement préférés sont les (méth)acrylates d'alkyle.

Comme polymère radicalaire, on utilise de préférence les copolymères d'acide (méth)acrylique et de (méth) acrylate d'alkyle, notamment d'alkyle en $C_1$-$C_4$. Plus préférentiellement, on peut utiliser les acrylates de méthyle éventuellement copolymérisés avec l'acide acrylique.

Comme amides des monomères acides, on peut citer les (méth)acrylamides, et notamment les N-alkyl (méth) acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase grasse peuvent représenter en matière sèche de 5 à 40% du poids de la composition, de préférence de 5 à 35 % et mieux de 8 à 30%.

Selon un mode de mise en oeuvre, les particules de polymère en dispersion sont stabilisées en surface par un stabilisant solide à température ambiante. Dans ce cas, la quantité en matière sèche de la dispersion représente la quantité totale de polymère + stabilisant, sachant que la quantité de polymère ne peut être inférieure à 5%.

Les particules de polymère sont de préférence stabilisées en surface grâce à un stabilisant, qui peut être un polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Le stabilisant peut être également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "Dow Corning 3225C" par la société Dow Corning, les lauryl méthicones tels que ceux vendu sous la dénomination "Dow Corning Q2-5200 par la société "Dow Corning".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylènepropylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de 'Kraton' par Shell Chemical Co ou encore du type polystyrène/copoly(éthylène-butylène) (SEB). En particulier, on peut utiliser le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS). Les polymères sont généralement appelés des copolymères de diènes hydrogénés ou non.

On peut aussi utiliser les Gelled Permethyl 99A-750, 99A-753-59 et 99A-753-58 (mélange de tribloc et de polymère en étoile), Versagel 5960 de chez Penreco (tribloc + polymère en étoile) ; OS129880, OS129881 et OS84383 de chez Lubrizol (copolymère styrène/méthacrylate).

Comme copolymères blocs greffés ou séquencés comprenant au moins un bloc résultant de la polymérisation d'au moins un monomère éthylénique à une

ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une

ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse du polymère est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le solvant de synthèse ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,
- (c) les copolymères blocs greffés ou séquencés comprenant au moins un bloc résultant de la polymérisation d'au

moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

**Dispersion aqueuse de particules polymères**

Selon un autre mode de réalisation, le polymère filmogène peut être choisi parmi les dispersions aqueuses de particules polymères, dans le cas où la composition selon l'invention comprend une phase aqueuse.

La dispersion aqueuse comprenant un ou plusieurs polymères filmogènes peut être préparée par l'homme de l'art sur la base de ses connaissances générales, en particulier par une polymérisation en émulsion ou par une mise en dispersion du polymère précédemment formé.

Parmi les polymères filmogènes pouvant être utilisés dans la composition selon la présente invention, on peut citer les polymères synthétiques du type polycondensat ou du type radical, les polymères d'origine naturelle, et des mélanges de ceux-ci.

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors de la polycondensation. Les résines d'époxyester peuvent être obtenues par la polycondensation d'acides gras avec un condensat au niveau des extrémités $\alpha,\omega$-diépoxy.

Les polymères radicalaires peuvent être en particulier les polymères ou les copolymères acryliques et/ou vinyliques. Les polymères à radical anionique sont préférés. Comme monomère portant un groupement anionique pouvant être utilisé lors de la polymérisation radicalaire, on peut citer l'acide acrylique, l'acide méthacrylique, l'acide crotonique, l'anhydride maléique et l'acide 2-acrylamido-2-méthylpropanesulfonique.

Les polymères acryliques peuvent résulter de la copolymérisation de monomères choisis parmi les esters et/ou les amides de l'acide acrylique ou de l'acide méthacrylique. Comme exemples de monomères du type ester, on peut

citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise de préférence les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/acrylique.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

Lorsqu'une dispersion aqueuse de particules polymères est utilisée, la teneur en matière sèche de ladite dispersion aqueuse peut être de l'ordre de 3 à 60% en poids, et préférablement de 10 à 50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

Comme dispersion aqueuse de polymère filmogène, on peut utiliser les dispersions acryliques vendues sous les dénominations « Neocryl XK-90® », « Neocryl A-1070® », « Neocryl A-1090® », « Neocryl BT-62® », « Neocryl A-1079® » et « Neocryl A-523® » par la société AVECIA-NEORESINS, « Dow Latex 432® » par la société DOW CHEMICAL, « Daitosol 5000 AD® » ou « Daitosol 5000 SJ » par la société DAITO KASEY KOGYO; « Syntran 5760 » par la société Interpolymer ou encore les dispersions aqueuses de polyuréthane vendues sous les dénominations « Neorez R-981® » et « Neorez R-974® » par la société AVECIA-NEORESINS, les « Avalure UR-405® », « Avalure UR-410® », « Avalure UR-425® », « Avalure UR-450® », « Sancure 875® », « Sancure 861® », « Sancure 878® » et « Sancure 2060® » par la société GOODRICH, « Impranil 85® » par la société BAYER, « Aquamere H-1511® » par la société HYDROMER ; les sulfopolyesters vendus sous le nom de marque « Eastman AQ® » par la société EASTMAN CHEMICAL PRODUCTS, les dispersions vinyliques comme le « Mexomère PAM », les dispersions aqueuses de polyvinyl acétate comme le « Vinybran® » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylmethacrylamidoammonium telles que le Styleze W-d'ISP, les dispersion aqueuse de polymère hybrides polyuréthane/polyacryliques telles que celles commercialisées sous les références « Hybridur® » par la société AIR PRODUCTS ou « Duromer® » de NATIONAL STARCH, les dispersions type core/shell : par exemple celles commercialisées par la société ATOFINA sous la référence Kynar (core : fluoré -shell : acrylique) ou encore ceux décrits dans le document US 5 188 899 (core : silice - shell : silicone) et leurs mélanges.

**Polymère filmogène hydrosoluble**

Dans le cas où la composition comprend une phase aqueuse, le polymère filmogène peut être un polymère hydrosoluble. Le polymère hydrosoluble est donc solubilisé dans la phase aqueuse de la composition.

Parmi les polymères filmogènes hydrosolubles, on peut citer les polymères cationiques suivants :

(1) les polymères ou copolymères acryliques, tels que les polyacrylates ou les polyméthacrylates ; les copolymères de la famille (1) peuvent contenir en outre un ou plusieurs motifs dérivant de comonomères pouvant être choisis parmi la famille des acrylamides, des méthacrylamides, des diacétoneacrylamides, des acrylamides et méthacrylamides substitués sur l'azote par des alkyles inférieurs, des acides acrylique ou méthacrylique ou des esters de ceux-ci, des vinyllactames tels que la vinylpyrrolidone ou le vinylcaprolactame, des esters vinyliques.
Ainsi, parmi ces copolymères de la famille (1), on peut citer :

- les copolymères d'acrylamide et de méthacrylate de diméthylaminoéthyle quaternisés par le sulfate de diméthyle, ou par un halogénure de diméthyle tel que celui commercialisé sous la dénomination HERCOFLOC par la société HERCULES,
- le copolymère d'acrylamide et de chlorure de méthacryloyloxyéthyltriméthylammonium décrit, par exemple, dans la demande de brevet EP-A-0 809 76 et commercialisé sous la dénomination BINA QUAT P 100 par la société CIBA GEIGY,
- le copolymère d'acrylamide et de méthosulfate de méthacryloyloxyéthyltriméthylammonium commercialisé sous

la dénomination RETEN par la société HERCULES,

- les copolymères de vinylpyrrolidone/acrylate ou méthacrylate de dialkylaminoalkyle, quaternisés ou non quaternisés, tels que les produits commercialisés sous la dénomination « GAFQUAT » par la société ISP, tels que par exemple « GAFQUAT 734 » ou « GAFQUAT 755 », ou bien les produits désignés par « COPOLYMER 845, 958 et 937 ». Ces polymères sont décrits en détail dans les brevets français 2 077 143 et 2 393 573,
- les terpolymères de méthacrylate de diméthyl-aminoéthyle/vinylcaprolactame/vinylpyrrolidone tels que le produit commercialisé sous la dénomination GAFFIX VC 713 par la société ISP, et
- le copolymère de vinylpyrrolidone/diméthylaminopropylméthacrylamide quaternisé tel que le produit commercialisé sous la dénomination « GAFQUAT HS 100 » par la société ISP.

(2) les polysaccharides quaternisés décrits plus particulièrement dans les brevets US 3 589 578 et US 4 031 307, tels que les gommes de guar contenant des groupements trialkylammonium cationiques. De tels produits sont commercialisés en particulier sous les dénominations commerciales JAGUAR C13 S, JAGUAR C 15 et JAGUAR C 17 par la société MEYHALL.
(3) les copolymères de vinylpyrrolidone et de vinylimidazole quaternaires ;
(4) les chitosanes ou les sels de ceux-ci ;
(5) les dérivés de cellulose cationiques, tels que les copolymères de cellulose ou de dérivés de cellulose greffés par un monomère hydrosoluble comprenant un ammonium quaternaire et décrits en particulier dans le brevet US 4 131 576, tels que les hydroalkyl celluloses, comme les hydroxyméthyl, hydroxyéthyl ou hydroxypropyl celluloses greffées en particulier par un sel de méthacryloyloxyéthyltriméthylammonium, de méthacrylamidopropyltriméthylammonium ou de diméthyldiallylammonium. Les produits commercialisés correspondant à cette définition sont plus particulièrement les produits commercialisés sous la dénomination « CELQUAT L 200 » et « CELQUAT H 100 » par la National Starch Company.

Parmi les polymères hydrosolubles filmogènes, on peut citer les polymères amphotères suivants :

(1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloroacrylique, et d'un monomère basique dérivé d'un composé vinylique substitué contenant au moins un atome de base tel que plus particulièrement un méthacrylate et acrylate de dialkylaminoalkyle, et un dialkylaminoalkylméthacrylamide et acrylamide. De tels composés sont décrits dans le brevet américain n° 3 836 537.
(2) les polymères comprenant des motifs dérivant :

a) d'au moins un monomère choisi parmi les acrylamides ou les méthacrylamides substitués sur l'azote par un radical alkyle,
b) d'au moins un comonomère acide contenant un ou plusieurs groupements carboxyliques réactifs, et
c) d'au moins un comonomère basique tel que les esters, ayant des substituants amine primaire, secondaire, tertiaire et quaternaire, d'acides acrylique et méthacrylique, et le produit de la quaternisation du méthacrylate de diméthylaminoéthyle par du sulfate de diméthyle ou de diéthyle.

(3) les alkoylpolyaminoamides réticulés dérivés totalement ou en partie de polyaminoamides.
(4) les polymères comprenant des motifs zwitterioniques.
(5) le polymère dérivé du chitosane.
(6) les polymères dérivés de la N-carboxyalkylation du chitosane, tels que le N-carboxyméthyl chitosane ou le N-carboxybutyl chitosane commercialisé sous la dénomination « EVALSAN » par la société JAN DEKKER.
(7) les copolymères du ($C_1$-$C_5$)alkylvinyléther/ anhydride maléique partiellement modifié par une semi-amidification par une N,N-dialkylaminoalkylamine, telle que la N,N-diméthylaminopropylamine ou par une semi-estérification par une N,N-dialcanolamine. Ces copolymères peuvent également comporter d'autres comonomères vinyliques tels que le vinylcaprolactame.

Les polymères filmogènes hydrosolubles sont préférablement choisis parmi le groupe constitué par :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;

- les polymères vinyliques, tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :
  - la gomme arabique, la gomme de guar, les dérivés du xanthane, la gomme de karaya ;
  - les alginates et les carraghénanes ;
  - les glycoaminoglycanes, l'acide hyaluronique et ses dérivés ;
  - la gomme laque, la gomme sandaraque, les dammars, l'élémis, les copals ;
  - l'acide désoxyribonucléique ;
  - les mucopolysaccharides, tels que l'acide hyaluronique, le sulfate de chondroïtine, et des mélanges de ceux-ci.

Ces polymères seront utilisés en particulier si l'on désire une élimination plus ou moins appréciable du film par de l'eau.

**Polymères ayant un squelette organique non siliconé greffé par des monomères contenant un polysiloxane**

Selon un mode de réalisation de l'invention, le polymère filmogène peut être choisi parmi les polymères ayant un squelette organique non siliconé greffé par des monomères contenant un polysiloxane. Ces polymères peuvent être liposolubles, lipodispersibles, hydrosolubles ou dispersibles en milieu aqueux, le cas échéant.

Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.

Dans ce qui suit, on doit comprendre que l'expression « macromère de polysiloxane » désigne, ainsi qu'il est généralement accepté, tout monomère contenant une chaîne polymère du type polysiloxane dans sa structure.

Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.

Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non siliconés, eux même correctement fonctionnalisés par une fonction qui est capable de réagir avec le groupement ou les groupements fonctionnel(s) porté(s) par ladite silicone en formant une liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.

Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un macromère de polysiloxane ayant une fonction terminale réactive avec lesdits groupements fonctionnalisés.

Une famille particulière de polymères siliconés greffés convenables pour la mise en oeuvre de la présente invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale :

$$X(Y)_nSi(R)_{3-m}Z_m \qquad (I)$$

dans laquelle :

X désigne un groupement vinyle copolymérisable avec les monomères (A) et (B) ;
Y désigne un groupement ayant une liaison divalente ;
R désigne hydrogène, alkyle ou alkoxy en $C_1$-$C_6$, aryle en $C_6$-$C_{12}$;
Z désigne un motif polysiloxane monovalent ayant un poids moléculaire moyen en nombre d'au moins 500 ;
n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.

Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique , le styrène ; les macromères de polystyrène ; l'acétate de vinyle ; le propionate de vinyle ; l'alpha-méthylstyrène ; le tertio-butylstyrène ; le butadiène ; le cyclohexadiène ; le cyclohexadiène [sic] ; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcool-fluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfonamido)éthyle, ou des mélanges de ceux-ci.

Comme exemples de monomères (B) polaires, on peut citer l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth) acrylates d'hydroxyalkyle, le chlorure de diallyldiméthylammonium, la vinylpyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.

On cite notamment le produit KP 561 ou le KP 562 commercialisé par Shin Etsu tel que le monomère (A) et choisi parmi les esters d'alcool en $C_{18}$-$C_{22}$ et de l'acide méthacrylique.

Les macromères de polysiloxane (C) de formule (I) sont choisis de préférence parmi ceux correspondant à la formule générale (II) suivante :

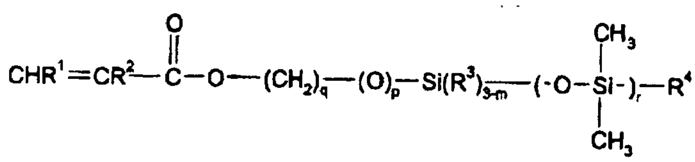

(II)

dans laquelle :

$R^1$ est hydrogène ou -COOH (préférablement hydrogène) ;
$R^2$ est hydrogène, méthyle ou $-CH_2COOH$ (préférablement méthyle) ;
$R^3$ est alkyle, alkoxy ou alkylamino en $C_1$-$C_6$, aryle en $C_6$-$C_{12}$ ou hydroxyle (préférablement méthyle) ;
$R^4$ est alkyle, alkoxy ou alkylamino en $C_1$-$C_6$, aryle en $C_6$-$C_{12}$ ou hydroxyle (préférablement méthyle) ;
q est un nombre entier allant de 2 à 6 (préférablement 3) ;
p vaut 0 ou 1 ;
r est un nombre entier allant de 5 à 700 ;
m est un nombre entier allant de 1 à 3 (préférablement 1).

On utilise de préférence les macromères de polysiloxane de formule :

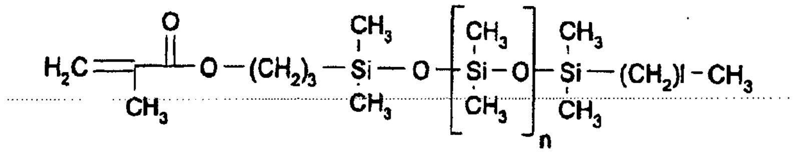

n étant un nombre allant de 5 à 700 et I étant un nombre entier compris entre 0 et 3.

Un mode de réalisation de l'invention consiste en l'utilisation d'un copolymère capable d'être obtenu par une polymérisation radicalaire à partir du mélange de monomères constitué par :

a) 60% en poids d'acrylate de tertio-butyle ;
b) 20% en poids d'acide acrylique ;
c) 20% en poids de macromère siliconé de formule :

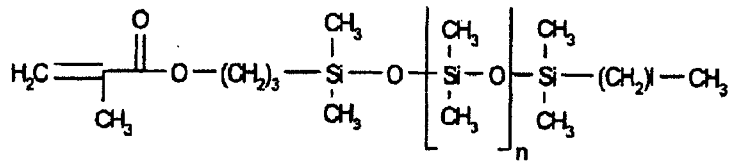

n étant un nombre allant de 5 à 700 et 1 étant un nombre entier compris entre 0 et 3, les pourcentages en poids étant calculés par rapport au poids total des monomères.

Un autre mode de réalisation particulier de l'invention consiste en l'utilisation d'un copolymère capable d'être obtenu par une polymérisation radicalaire à partir du mélange de monomères constitué par :

a) 80% en poids d'acrylate de tertio-butyle ;
b) 20% en poids de macromère siliconé de formule :

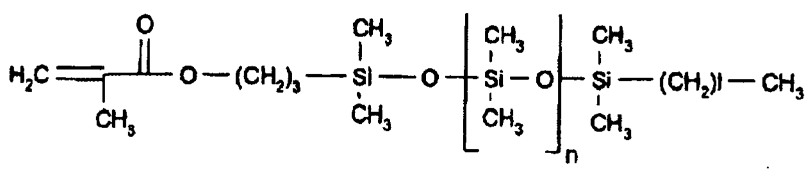

n étant un nombre allant de 5 à 700 et I étant un nombre entier compris entre 0 et 3, les pourcentages en poids étant calculés par rapport au poids total des monomères.

Une autre famille particulière de polymères siliconés greffés ayant un squelette organique non siliconé convenable pour une mise en oeuvre de la présente invention est constituée par les copolymères siliconés greffés capables d'être obtenus par l'extrusion réactive d'un macromère de polysiloxane à fonction terminale réactive sur un polymère du type polyoléfine comprenant des groupements réactifs capables de réagir avec la fonction terminale du macromère de polysiloxane pour former une liaison covalente permettant le greffage de la silicone sur la chaîne principale de la polyoléfine. Ces polymères, ainsi que leur procédé de préparation, sont décrits dans la demande de brevet WO 95/00578.

Les polyoléfines réactives sont choisies de préférence parmi les polyéthylènes ou les polymères de monomères dérivés de l'éthylène, tels que le propylène, le styrène, l'alkylstyrène, le butylène, le butadiène, les (méth)acrylates, les esters vinyliques ou équivalents, comprenant des fonctions réactives capables de réagir avec la fonction terminale du macromère de polysiloxane. Ils sont choisis plus particulièrement parmi les copolymères d'éthylène ou de dérivés d'éthylène et les monomères choisis parmi ceux comprenant une fonction carboxylique tels que l'acide (méth)acrylique ; ceux comprenant une fonction anhydride d'acide tels que l'anhydride de l'acide maléique ; ceux comprenant une fonction chlorure d'acide tels que le chlorure de l'acide (méth)acrylique ; ceux comprenant une fonction ester tels que les esters de l'acide (méth)acrylique ; et ceux comportant une fonction isocyanate.

Les macromères siliconés sont choisis de préférence parmi les polysiloxanes comprenant un groupement fonctionnalisé, à l'extrémité de la chaîne de polysiloxane ou à proximité de l'extrémité de ladite chaîne, choisis au sein du groupe constitué par les alcools, les thiols, les époxy, les amines primaires et secondaires, et tout particulièrement parmi ceux correspondant à la formule générale :

$$T\text{-}(CH_2)_6\text{-}Si\text{-}[\text{-}(OSiR^5R^6)_t\text{-}R^7]_y \qquad (III)$$

dans laquelle T est choisi parmi le groupe constitué par $NH_2$, NHRN, une fonction époxy, OH, SH ; $R^5$, $R^6$, $R^7$ et RN, indépendamment, désignent alkyle en $C_1$-$C_6$, phényle, benzyle ou alkylphényle en $C_6$-$C_{12}$, hydrogène ; s est un nombre allant de 2 à 00 [sic], t est un nombre allant de 0 à 1000 et y est un nombre allant de 1 à 3. Ils ont un poids moléculaire moyen en nombre allant préférablement de 5000 à 300 000, plus préférablement de 8000 à 200 000, et plus particulièrement de 9000 à 40 000.

Selon un mode de réalisation préféré, le polymère filmogène peut être acheté auprès de la Minnesota Mining and Manufacturing Company sous les dénominations commerciales de polymères « Silicone Plus ». Par exemple, le poly(méthacrylate d'isobutyle-co-FOSEA de méthyle)-g-poly(diméthylsiloxane) est commercialisé sous la dénomination commerciale SA 70-5 IBMMF.

Selon une autre forme préférée de l'invention, le polymère filmogène est choisi parmi les polymères siliconés greffés par des monomères organiques non siliconés. Ces polymères peuvent être liposolubles, lipodispersables, hydrosolubles ou dispersables en milieu aqueux, le cas échéant.

Ledit polymère ou lesdits polymères siliconé(s) greffé(s) ayant un squelette de polysiloxane greffé par des monomères organiques non siliconés contenant une chaîne principale de silicone (ou de polysiloxane $(/SiO\text{-})_n$) sur laquelle se trouve greffé, à l'intérieur de ladite chaîne ainsi qu'éventuellement à l'une au moins de ses extrémités, au moins un groupement organique ne comprenant pas de silicone.

Les polymères ayant un squelette de polysiloxane greffé par des monomères organiques non siliconés selon l'invention peuvent être des produits commerciaux existants ou bien ils peuvent être obtenus par tout moyen connu de l'homme de l'art, en particulier par une réaction entre (i) une silicone de départ correctement fonctionnalisée sur un ou plusieurs de ces atomes de silicium et (ii) un composé organique non siliconé lui-même correctement fonctionnalisé par une fonction qui est capable de réagir avec le groupement ou les groupements fonctionnel(s) porté(s) par ladite silicone en formant une liaison covalente ; un exemple classique d'une telle réaction est la réaction d'hydrosylilation entre des groupements /Si-H et des groupements vinyliques $CH_2$=CH-, voire la réaction entre des groupements thiofonctionnels -SH avec ces mêmes groupements vinyle.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776, dont les enseignements sont totalement inclus dans la présente description à titre de références non limitatives.

Selon un mode de réalisation particulièrement préféré de la présente invention, le polymère siliconé, ayant un squelette de polysiloxane greffé par des monomères organiques non siliconés, mis en oeuvre, est constitué du résultat d'une copolymérisation radicalaire entre, d'une part, au moins un monomère organique anionique non siliconé à insaturation éthylénique et/ou un monomère organique hydrophobe non siliconé à insaturation éthylénique et, d'autre part, une silicone présentant dans sa chaîne au moins un groupement fonctionnel, et préférablement plusieurs, capable de réagir avec lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou comme mélanges, parmi les acides carboxyliques insaturés, linéaires ou ramifiés, éventuellement neutralisés partiellement ou totalement sous forme d'un sel, ce ou ces acide(s) carboxylique(s) insaturé(s) pouvant être plus particulièrement l'acide acrylique, l'acide méthacrylique, l'acide maléique, l'acide itaconique, l'acide fumarique et l'acide crotonique. Les sels convenables sont en particulier les sels alcalins, alcalino-terreux et d'ammonium. On notera que, de même, dans le polymère siliconé greffé final, le groupement organique de nature anionique qui est constitué du résultat de l'(homo)polymérisation radicalaire d'au moins un monomère anionique du type acide carboxylique insaturé peut être, après réaction, post-neutralisé par une base (soude, ammoniaque, etc.) pour l'amener sous la forme d'un sel.

Selon la présente invention, les monomères hydrophobes à insaturation éthylénique sont de préférence choisis, seuls ou comme mélange, parmi les esters de l'acide acrylique d'alcanols et/ou les esters de l'acide méthacrylique d'alcanols. Les alcanols sont de préférence en $C_1$-$C_{30}$ et plus particulièrement en $C_1$-$C_{22}$. Les monomères préférés sont choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle et le (méth)acrylate de stéaryle, ou des mélanges de ceux-ci.

Une famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule IV ci-dessous :

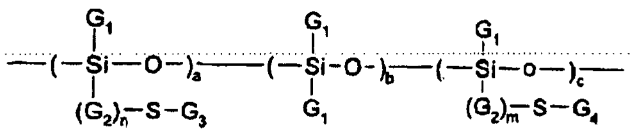

(IV)

dans laquelle les radicaux $G_1$, identiques ou différents, représentent hydrogène ou un radical alkyle en $C_1$-$C_{10}$, voire un radical phényle ; les radicaux $G_2$, identiques ou différents, représentent représente [sic] un groupement alkylène en $C_1$-$C_{10}$; $G_3$ représente un reste polymère résultant de l'(homo)polymérisation d'au moins un monomère anionique à insaturation éthylénique ; $G_4$ représente un reste polymère résultant de l'(homo)polymérisation d'au moins un monomère d'au moins un monomère hydrophobe [sic] à insaturation éthylénique ; m et n sont égaux à 0 ou 1 ; a est un nombre entier allant de 0 à 50 ; b est un nombre entier pouvant être compris entre 10 et 350, c est un nombre entier allant de 0 à 50 ; à condition que l'un des paramètres a et c soit différent de 0.

Le motif de formule (IV) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène ;
- $G_3$ représente un radical polymère résultant de l'(homo)polymérisation d'au moins un monomère du type acide carboxylique à insaturation éthylénique, préférablement l'acide acrylique et/ou l'acide méthacrylique ;
- $G_4$ représente un radical polymère résultant de l'(homo)polymérisation d'au moins un monomère du type (méth) acrylate d'alkyle en $C_1$-$C_{10}$, préférablement le (méth)acrylate d'isobutyle ou de méthyle.

Des exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères mixtes du type acide poly(méth)acrylique et du type poly(méth)acrylate d'alkyle.

D'autres exemples de polymères siliconés correspondant à la formule (IV) sont en particulier des polydiméthylsiloxanes (PDMS) sur lesquels sont greffés, par l'intermédiaire d'une liaison secondaire du type thiopropylène, des motifs polymères du type poly(méth)acrylate d'isobutyle.

De tels polymères comportent les polymères comprenant au moins un groupement de formule :

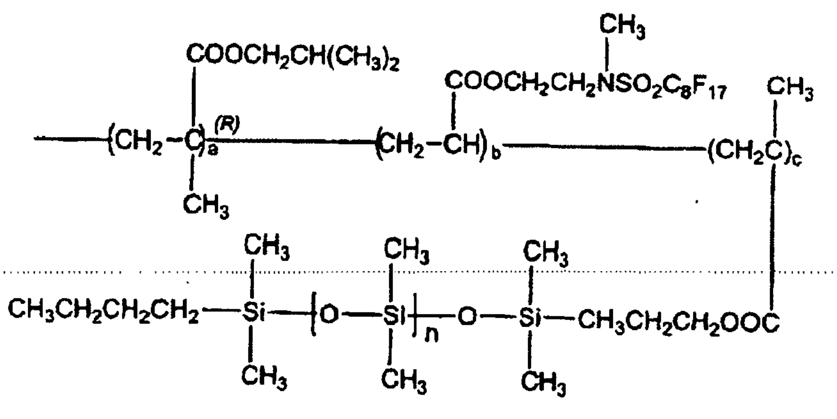

dans laquelle
a, b et c, pouvant être identiques ou différents, sont chacun un nombre allant de 1 à 100 000 ; et les groupements terminaux, pouvant être identiques ou différents, sont choisis chacun parmi les groupements alkyle linéaires en $C_1$-$C_{20}$, les groupements alkyle à chaîne ramifiée en $C_3$-$C_{20}$, les groupements aryle en $C_3$-$C_{20}$, les groupements alkoxy linéaires en $C_1$-$C_{20}$ et les groupements alkoxy ramifiés en $C_3$-$C_{20}$.

De tels polymères sont divulgués dans les brevets US n° 4 972 037, 5 061 481, 5 209 924, 5 849 275 et 6 033 650, et WO 93/23446 et WO 95/06078.

Une autre famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (V) ci-dessous :

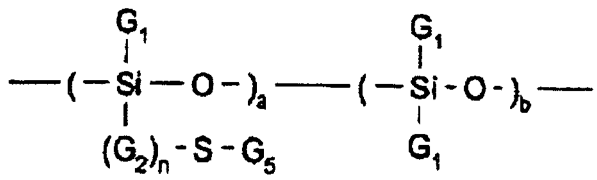

(V)

dans laquelle les radicaux $G_1$ et $G_2$ ont la même signification que ci-dessus; $G_5$ représente un reste polymère résultant de l'(homo)polymérisation d'au moins un monomère d'au moins un monomère hydrophobe [sic] à insaturation éthylénique ou de la copolymérisation d'au moins un monomère anionique à insaturation éthylénique et d'au moins un monomère hydrophobe à insaturation éthylénique ; n est égal à 0 ou 1 ; a est un nombre entier allant de 0 à 50 ; b est un nombre entier pouvant être compris entre 10 et 350 ; à condition que a soit différent de 0.

Le motif de formule (V) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène.

La masse moléculaire en nombre des polymères siliconés ayant un squelette de polysiloxane greffé par des monomères organiques non siliconés de l'invention varie préférablement d'environ 10 000 à 1 000 000, et encore plus préférablement d'environ 10 000 à 100 000.

La composition peut contenir de 0,5 à 60% en poids, mieux de 1 à 40%, préférablement de 2 à 30% en poids de matière sèche d'agent filmogène par rapport au poids total de la composition.

Plus généralement, la quantité totale de polymère doit être en quantité suffisante pour former sur la peau et/ou les lèvres un film cohésif capable de suivre les mouvements de la peau et/ou des lèvres sans se décoller ou craquer.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

<u>PATEUX</u>

La composition peut également contenir en outre au moins un composé pâteux.

Le composition est avantageusement exempte de lanoline ou de dérivés de lanoline.

A titre illustratif des dérivés de lanoline utilisés conventionnellement, on peut notamment citer la lanoline liquide, la lanoline réduite, la lanoline purifiée par adsorption, la lanoline acétylée, la cire de lanoline oxypropylénée (5 OP), l'acétate de lanoline liquide, l'hydroxylanoline, la polyoxyéthylène-lanoline, l'acide gras de lanoline, l'acide gras de lanoline dure, les esters de cholestéryle d'acide gras de lanoline, l'alcool de lanoline, l'acétate de l'alcool de lanoline, le lanolate d'isopropyle et autres.

Les lanolines présentent l'inconvénient d'être sensibles à la chaleur et aux ultraviolets. Elles ont tendance à s'oxyder avec un dégagement d'odeur désagréable, et leur couleur très jaune empêche de les utiliser dans des bases de soin non pigmentées et les bases incolores, ce qui limite leur utilisation dans les compositions cosmétiques.

Le demandeur a découvert que les esters alcoxylés décrits précédemment sont de bons substituts de la lanoline ou de ses dérivés.

Par "pâteux" au sens de la présente invention, on entend désigner un composé gras lipophile, à changement d'état solide/liquide réversible, et comportant à la température de 23°C une fraction liquide et une fraction solide. On entend également par « pâteux », le polylaurate de vinyle.

Le composé pâteux est avantageusement choisi parmi :

- la lanoline et ses dérivés,
- les composés fluorés polymères ou non,
- les composés siliconés polymères ou non,
- les polymères vinyliques, notamment:
    - les homopolymères d'oléfines
    - les copolymères d'oléfines
    - les homopolymères et copolymères de diènes hydrogénés

- les oligomères linéaires ou ramifiés, homo ou copolymères de (méth)acrylates d'alkyles ayant de préférence un groupement alkyle en $C_8$-$C_{30}$
- les oligomères homo et copolymères d'esters vinyliques ayant des groupements alkyles en $C_8$-$C_{30}$
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters,
  et leurs mélanges.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyethylène/polydodécyle glycol tel que les éthers de dodécanediol (22 mol) et de polyéthylène glycol (45 OE) commercialisés sous la marque ELFACOS ST9 par Akzo Nobel.
Parmi les pâteux esters, on préfère notamment :

- les esters d'un glycérol oligomère, notamment les esters de diglycérol, en particulier les condensats d'acide adipique et de glycérol, pour lesquels une partie des groupes hydroxyle des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les polyesters non réticulés résultant de la polycondensation entre un diacide ou un polyacide carboxylique linéaire ou ramifié en $C_4$-$C_{50}$ et un diol ou un polyol en $C_2$-$C_{50}$, différent du polyester décrit précédemment,
- les esters aliphatiques d'ester résultant de l'estérification d'un ester d'acide hydroxycarboxylique aliphatique par un acide monocarboxylique aliphatique; et leurs mélanges, comme

  - l'ester résultant de la réaction d'estérification de l'huile de ricin hydrogénée avec l'acide isostéarique dans les proportions 1 pour 1 (1/1) ou monoisostéarate d'huile de ricin hydrogénée,
  - l'ester résultant de la réaction d'estérification de l'huile de ricin hydrogénée avec l'acide isostéarique dans les proportions 1 pour 2 (1/2) ou le diisostéarate d'huile de ricin hydrogénée,
  - l'ester résultant de la réaction d'estérification de l'huile de ricin hydrogénée avec l'acide isostéarique dans les proportions 1 pour 3 (1/3) ou trüsostéarate d'huile de ricin hydrogénée,
  - et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.
Le composé pâteux représente de préférence 1 à 99%, mieux 1 à 60%, mieux 2 à 30% et mieux encore 5 à 15% en poids de la composition.

COLORANT

Avantageusement, la composition de l'invention peut comprendre, en outre, au moins une matière colorante qui peut être choisie parmi les colorants, les pigments, les nacres et leurs mélanges. Cette matière colorante peut représenter de 0,001 à 98 %, de préférence de 0,5 à 85% et mieux de 1 à 60 % du poids total de la composition.
Les colorants sont de préférence des colorants liposolubles, bien que les colorants hydrosolubles puissent être utilisés. Les colorants liposolubles sont par exemple le rouge Soudan, le D & C Red 17, le D & C Green 6, le β-carotène, l'huile de soja, le brun Soudan, le D & C Yellow 11, le D & C Violet 2, le D & C orange 5, le jaune quinoléine, le rocou. Ils peuvent représenter de 0 à 20 % du poids de la composition et mieux de 0,1 à 6 %. Les colorants hydrosolubles sont notamment le jus de betterave, le bleu de méthylène et peuvent représenter de 0,1 à 6 % en poids de la composition (si présents).
Pour une composition sous forme de pâte ou coulée telle que les rouges à lèvres ou les produits de maquillage du corps, on utilise en général de 0,5 à 50% de matière colorante, de préférence de 2 à 40 % et mieux de 5 à 30%, par rapport au poids total de la composition.
Par pigments, il faut comprendre des particules blanches ou colorées, minérales ou organiques, insolubles dans la phase grasse liquide, destinées à colorer et/ou opacifier la composition. Par charges, il faut comprendre des

particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments peuvent être présents dans la composition à raison de 0,05 à 30 % du poids de la composition finale, et de préférence à raison de 2 à 20 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium (D & C Red N°7), aluminium.

Les nacres peuvent être présentes dans la composition à raison de 0,001 à 20 % du poids total de la composition, de préférence à un taux de l'ordre de 1 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré. La composition contient avantageusement des pigments goniochromatiques, par exemple des pigments multicouches interférentiels, et/ou des pigments réfléchissants. Ces deux types de pigments sont décrits dans la demande FR0209246 dont le contenu est incorporé par référence dans la présente demande.

CHARGES

La composition peut contenir au moins une charge qui peut être présente à raison de 0,001 à 35 % du poids total de la composition, de préférence 0,5 à 15 %.

On peut notamment citer

- le talc, le mica, le kaolin, l'amidon
- les poudres de Nylon® (Orgasol notamment)
- les poudres de polyéthylène,
- les poudres de polytétrafluoroéthylène (Téflon®),
- le nitrure de bore,
- des microsphères de copolymères telles que l'Expancel® (Nobel Industrie),
- le Polytrap® 603 (Dow Corning),
- le Polypore® L 200 (Chemdal Corporation)
- les microbilles de résine de silicone (Tospearl ® de Toshiba, par exemple),
- les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass
- les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

La charge peut être par exemple une charge dont la granulométrie moyenne est inférieure à 100 μm, notamment comprise entre 1 et 50 μm, par exemple entre 4 et 20 μm. La charge peut être de toute forme, essentiellement sphérique ou sous la forme de plaquettes.

CIRE

La composition peut contenir, en outre, au moins une cire. Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200° C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du

Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

La composition selon l'invention contient avantageusement de la cire de polyéthylène de masse moléculaire en poids comprise entre 300 et 700, notamment égale à 500 g/mol.

A titre indicatif, la cire peut représenter de 0,01 à 50 %, de préférence de 2 à 40 %, et mieux de 5 à 30 % du poids total de la composition.

HUILE NON VOLATILE

La composition peut également comprendre en outre au moins une huile non volatile différente de l'ester d'alcool alcoxylé. L'huile non volatile peut être choisie parmi:

- les huiles hydrocarbonées d'origine animale telles que le perhydrosqualène ;
- les huiles hydrocarbonées végétales telles que les triglycérides liquides d'acides gras ayant de 4 à 10 atomes de carbone comme les triglycérides des acides heptanoïque ou octanoïque ou l'huile de jojoba ;
- les hydrocarbures linéaires ou ramifiés, d'origine minérale ou synthétique tels que les huiles de paraffine et leurs dérivés, la vaseline ;
- les esters hydrocarbonés de formule RCOOR' dans laquelle RCOO représente un reste d'acide carboxylique comportant de 2 à 30 atomes de carbone, et R' représente une chaîne hydrocarbonée contenant de 1 à 30 atomes de carbone, tel que l'isononanoate d'isononyle, l'érucate d'oléyle ou le néopentanoate d'octyl-2-docécyle ;
- les alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées ;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones (telles que la phényl triméthicone vendue sous le nom commercial DC556 par Dow Corning), les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- les acides gras ayant de 12 à 26 atomes de carbone comme l'acide oléïque
- les huiles hydroxylées,
- et leurs mélanges.

Huile non volatile de masse moléculaire élevée

Selon un mode de réalisation, la composition contient une huile non volatile de masse moléculaire élevée, par exemple comprise entre 650 à 10000 g/mol.

La composition selon l'invention contient avantageusement de 2 à 30 %, de préférence de 5 à 25%, de 5 à 15% d'au moins une huile de masse molaire allant de 650 à 10000 g/mol, et de préférence allant de 900 et 7500 g/mol.

Ainsi, l'huile de masse moléculaire allant de 650 à 10000 g/mol peut être choisie parmi

- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
- les polydécènes et les polydécènes hydrogénés tels que le PURESYN 150 (MM=9200 g/mol) commercialisé par la société MOBIL CHEMICALS,
- les copolymères de la vinylpyrrolidone tels que le copolymère vinylpyrrolidone/1-héxadécène, ANTARON V-216 commercialisé ou fabriqué par la société ISP (MM=7300 g/mol),
- les esters tels que :

a) les esters d'acides gras linéaires ayant un nombre total de carbone allant de 35 à 70 comme le tétrapélargonate

de pentaérythrityle (MM=697,05 g/mol),
b) les esters hydroxylés tels que le triisostéarate de polyglycérol-2 (MM=965,58 g/mol),
c) les esters aromatiques tels que le tridécyl trimellitate (MM=757,19 g/mol),
d) les esters d'alcool gras ou d'acides gras ramifiés en $C_{24}$-$C_{28}$ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
e) les esters et polyesters de dimère diol, tels que les esters de dimère diol et d'acide gras , et les esters de dimère diols et de diacide.
Les esters de dimère diol et d'acide mono-carboxylique peuvent être obtenus à partir d'acide mono-carboxylique comprenant de 4 à 34 atomes de carbone notamment de 10 à 32 atomes de carbone, lesquels acides sont linéaires, ramifiés, saturés ou insaturés.
A titre illustratif des exemples d'acide mono-carboxylique convenant à l'invention, on peut notamment citer les acides gras.
Les esters de dimère diol et d'acide dicarboxylique peuvent être obtenus à partir d'un dimère diacide dérivé en particulier de la dimérisation d'un acide gras insaturé notamment en $C_8$ à $C_{34}$, notamment en $C_{12}$ à $C_{22}$, en particulier en $C_{16}$ à $C_{20}$, et plus particulièrement en $C_{18}$.
Selon une variante particulière, il s'agit plus particulièrement du dimère diacide dont dérive également le dimère diol à estérifier.
Les esters de dimère diol peuvent être obtenus à partir d'un dimère diol produit par hydrogénation catalytique d'un dimère diacide tel que décrit précédemment, par exemple le diacide dilinoléique hydrogéné.
A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7® .

- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

Les huiles non volatiles peuvent représenter de 0,001 à 90 % du poids total de la composition, de préférence de 0,05 à 60 % et mieux de 1 à 35 %.

HUILE VOLATILE

On peut inclure une ou plusieurs huiles volatiles dans la composition.
Par "huile volatile", on entend une huile (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm de Hg), et de préférence allant de 1,3 Pa à 13 000 Pa (0,01 à 100 mm de Hg), et préférentiellement allant de 1,3 Pa à 1 300 Pa (0,1 à 10 mm de Hg) .
En outre, l'huile volatile a généralement un point d'ébullition, mesuré à pression atmosphérique, allant de 150 ˚C à 260 ˚C, et de préférence allant de 170 ˚C à 250 ˚C.
Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor; elle peut contenir des groupes ester, éther, amine, amide.
Par huile siliconée, on entend une huile contenant au moins un atome de silicium, et notamment contenant des groupes Si-O.
Par huile fluorée, on entend une huile contenant au moins un atome de fluor.
L'huile volatile peut être siliconée ou hydrocarbonée.
L'huile volatile siliconée utilisable dans l'invention peut être choisie parmi les huiles siliconées ayant un point éclair allant de 40 ˚C à 102 ˚C, de préférence ayant un point éclair supérieur à 55 ˚C et inférieur ou égal à 95 ˚C, et préférentiellement allant de 65 ˚C à 95 ˚C.
Comme huiles siliconées volatiles utilisables dans l'invention, on peut citer les silicones linéaires ou cycliques ayant une viscosité à température ambiante inférieure à 8 cSt et ayant notamment de 2 à 7 atomes de silicium, ces

silicones comportant éventuellement des groupes alkyle ou alkoxy ayant de 1 à 10 atomes de carbone. Comme huile de silicone volatile utilisable dans l'invention, on peut citer notamment l'octaméthyl-cyclotétrasiloxane, le décaméthylcyclopentasiloxane, le dodécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Comme huiles siliconées volatiles utilisables dans l'invention, on peut citer les silicones décrites dans la demande FR0304259 non publiée.

L'huile volatile hydrocarbonée utilisable dans l'invention peut être choisie parmi les huiles hydrocarbonées ayant un point éclair allant de 40 ˚C à 102 ˚C, de préférence allant de 40 ˚C à 55 ˚C, et préférentiellement allant de 40 ˚C à 50 ˚C.

Comme huile volatile hydrocarbonée, on peut citer les huiles volatiles hydrocarbonées ayant de 8 à 16 atomes de carbone et leurs mélanges, et notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'iso-hexyle, et leurs mélanges. De préférence, l'huile volatile hydrocarbonée est choisie parmi les huiles volatiles hydrocarbonées ayant de 8 à 16 atomes de carbone et leurs mélanges, en particulier parmi l'isododécane, l'isodécane, l'isohexadécane, et est notamment l'isododécane.

L'huile volatile représente par exemple de 5 à 97,5 % du poids total de la composition, et mieux de 10 à 75 %, de préférence entre 20 et 50% du poids total de la composition.

L'huile volatile représente de préférence 20 à 50 % en poids de la composition, de préférence de 30 à 40%, de préférence 35% environ.

ADDITIFS

La composition de l'invention peut comprendre, en outre, tout additif complémentaire usuellement utilisé dans le domaine cosmétique, tel que de l'eau, des antioxydants, des conservateurs, des neutralisants, des plastifiants, des gélifiants lipophiles ou des composés non aqueux liquides, des gélifiants de phase aqueuse, des dispersants, des actifs cosmétiques. Ces additifs, à l'exception de l'eau qui peut représenter de 0 à 70 % et par exemple de 1 à 50 et mieux de 1 à 10 % du poids total de la composition, peuvent être présents dans la composition à raison de 0,0005 à 20% du poids total de la composition et mieux de 0,001 à 10%.

Comme actif cosmétique utilisable dans l'invention, on peut citer les vitamines A, E, C, $B_3$, F, les provitamines comme le D-panthénol, la glycérine, les actifs apaisants comme l'$\alpha$-bisabolol, l'aloe vera, l'allantoïne, les extraits de plantes ou les huiles essentielles, les agents protecteurs ou restructurants comme les céramides, les actifs "fraîcheur" comme le menthol et ses dérivés, les émollients (beurre de cacao, diméthicone), les hydratants (arginine PCA), les actifs antirides, les acides gras essentiels, les filtres solaires, et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

GALENIQUES

Les applications des compositions selon l'invention sont multiples et concernent l'ensemble des produits cosmétiques colorés ou non et plus particulièrement les rouges à lèvres.

La composition de l'invention peut se présenter sous la forme de composition solide, compactée ou coulée notamment en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

Elle peut se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. De préférence, elle se présente sous forme de composition à phase continue huileuse et notamment anhydre ; dans ce cas, elle peut contenir une phase aqueuse à un taux inférieur à 5 %.

La composition selon l'invention peut se présenter sous la forme d'une composition, colorée ou non, de soin de la peau, sous forme d'une composition de protection solaire ou de démaquillage ou encore sous forme d'une composition hygiénique. Si elle contient des actifs cosmétiques, elle peut alors être utilisée comme base de soin ou de traitement non thérapeutique pour la peau comme les mains ou le visage ou pour les lèvres (baumes à lèvres, protégeant les lèvres du froid et/ou du soleil et/ou du vent), produit de bronzage artificiel de la peau.

La composition de l'invention peut également se présenter sous la forme d'un produit de maquillage coloré de la peau, en particulier du visage comme un blush, un fard à joues ou à paupières, de maquillage du corps comme un produit de tatouage semi-permanent ou de maquillage des lèvres comme un rouge ou un brillant à lèvres, présentant

éventuellement des propriétés de soin ou de traitement non thérapeutique, un produit de maquillage des phanères comme par exemple un vernis à ongles, un mascara, un eyeliner, un produit de coloration ou de soin des cheveux.

De préférence, la composition selon l'invention se présente sous forme d'un rouge à lèvres ou d'un brillant à lèvres.

Bien entendu la composition de l'invention doit être physiologiquement acceptable (en particulier cosmétiquement acceptable), à savoir non toxique et susceptible d'être appliquée sur la peau, les phanères ou les lèvres d'êtres humains. Par « cosmétiquement acceptable », on entend agréable de goût, de toucher, d'aspect et/ou d'odeur, applicable plusieurs jours pendant plusieurs mois.

La composition selon l'invention peut être fabriquée par les procédés connus, généralement utilisés dans le domaine cosmétique.

Les exemples qui suivent ont pour but d'illustrer de manière non limitative l'objet de la présente invention. Les quantités sont données en pourcentage massique.

**Exemple 1 : Stick de rouge à lèvres**

| phase | Nom INCI | Nom commercial | % |
|---|---|---|---|
| A huile | Octyldodecyle PPG-3 myristyle ether dimer dilinoleate<br>Huile végétale hydrogénée | Liquiwax poly EFA<br>Cegesoft HF 52 | 23<br>5 |
| C cires | Cire alcool<br>Cire de polyethylene | Performacol 550 Alcohol<br>Polywax 500 | 3.25<br>12 |
| D | Pâtes pigmentaires | | 15.25 |
| E | VP/Eicosene copolymère | Antaron V220 | 2 |
| F | Sucrose acetate isobutyrate | Eastman SAIB special | 6 |
| G gel | Isododecane (and) Acrylate Copolymer (and) Ethylene/Propylene/ Styrene Copolymer<br>Cyclopentasiloxane (and) Diphenyl Dimethicone | Dispersion de polymère*<br>Mirasil C-DPDM | 9<br>1 |
| H | Isododecane (and) Acrylate Copolymer(and) Ethylene/Propylene/ Styrene Copolymer | Dispersion de polymère* | 23 |
| I | parfum | | 0.5 |

*Préparation de la dispersion de polymère On prépare une dispersion de copolymère non réticulé d'acrylate de méthyle et d'acide acrylique dans un rapport 95/5, dans l'isododécane, selon la méthode de l'exemple 1 du document EP-A-749 746, en remplaçant l'heptane par l'isododécane. On obtient ainsi une dispersion de particules de poly (acrylate de méthyle/acide acrylique) stabilisées en surface dans l'isododécane par un copolymère dibloc séquencé polystyrène/copoly(éhylène-propylène) vendu sous le nom de Kraton G1701, ayant un taux de matière sèche de 25% en poids.

**Mode opératoire :**

Dans un Discontimill on prépare la pâte pigmentaire avec du polyisobutène hydrogéné et l'acide polyhydroxystéarique. Le broyage dure environ 30 min.

La phase gel est préparée dans un poêlon double paroi sous Rayneri à froid pendant 30 min.

Dans un poêlon à double paroi, on pèse les cires, la pâte pigmentaire (phase D), la phase E et F. Le mélange est chauffé jusqu'à fonte totale des cires et complètement homogénéisé sous pale défloculeuse. On ajoute alors la phase A, G , H et I et la pâte est coulée à 95°C dans un moule à 40-42°C, placée au congélateur pendant 30 min (pour 200g de pâte) avant d'être conditionnée sous forme de stylo.

## Revendications

1. Composition cosmétique comprenant au moins un ester d'alcool alcoxylé et d'acide carboxylique et au moins un

polymère filmogène non cristallin solide à température ambiante, **caractérisée en ce que** l'ester alcoxylé est choisi parmi les composés de formule développée suivante :

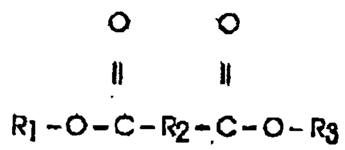

dans laquelle $R_1$ a la formule développée :

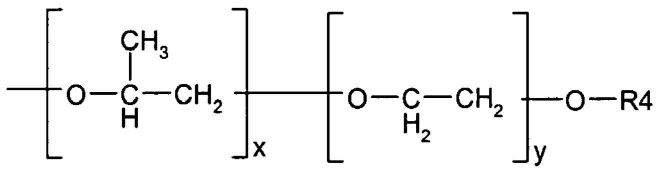

dans laquelle :

$R_4$ est un motif aliphatique saturé ou insaturé, substitué ou non, contenant de 4 à 24 atomes de carbone ;
x est un entier de 3 à 30 ; y est un entier de 3 à 30
$R_2$ est un motif aliphatique saturé ou insaturé, substitué ou non, contenant de 4 à 40 atomes de carbone ; et
$R_3$ est un motif aliphatique à chaîne droite ou à chaîne ramifiée, saturé ou insaturé, contenant de 4 à 32 atomes de carbone, notamment de 12 à 24 atomes de carbone.

2. Composition selon la revendication 1, **caractérisé en ce que** $R_4$ est un motif aliphatique saturé de 12 ou 20 atomes de carbone.

3. Composition selon la revendication 1 ou 2, **caractérisé en ce que** $R_2$ est un motif aliphatique saturé contenant de 4 à 40 atomes de carbone.

4. Composition selon la revendication 1, **caractérisé en ce que** x ou y sont indépendamment l'un de l'autre égaux à 3 ou 4.

5. Composition selon la revendication 1, **caractérisé en ce que** $R_3$ est un motif aliphatique saturé à chaîne ramifiée, contenant de 12 à 20 atomes de carbone.

6. Composition selon la revendication 4, **caractérisé en ce que** $R_3$ est choisi parmi l'octyldodécyle, l'isostéaryle et le stéaryle.

7. Composition selon la revendication 1, **caractérisé en ce que** l'ester alcoxylé est l'Octyldodécyl PPG-3 Myristyl Ether Dimer Dilinoléate.

8. Composition selon la revendication 1, **caractérisé en ce que** l'ester alcoxylé est l'Isostéaryl PPG-4 Butyloctyl Ether Dimer Dilinoléate.

9. Composition selon l'une des revendications précédentes, **caractérisée en ce que** le polymère est un polymère filmogène qui est tel, que lorsqu'il est en quantité suffisante dans la composition, ladite composition est apte à former un dépôt dont la tenue est supérieure ou égale à 30%.

10. Composition selon l'une quelconque des revendications précédentes, **caractérisée en ce que** le polymère filmogène est choisi parmi les gommes de silicone.

11. Composition selon l'une quelconque des revendications 1 à 10, **caractérisée en ce que** le polymère est choisi

parmi les résines de silicone.

**12.** Composition selon l'une selon l'une quelconque des revendications 1 à 10, **caractérisée en ce que** le polymère est choisi parmi les copolymères acryliques blocs ou radicalaires, les polymères acryliques greffés par un macromonomère siliconé, et leurs mélanges.

**13.** Composition selon l'une quelconque des revendications 1 à 10, **caractérisée en ce que** le polymère est un polyisoprène.

**14.** Composition selon l'une quelconque des revendications 1 à 10, **caractérisée en ce que** le polymère est un copolymère polyamide/polysiloxane.

**15.** Composition selon la revendication précédente, **caractérisée par le fait que** la composition est apte à former un dépôt ayant un indice de tenue supérieur ou égal à 40 %.

**16.** Composition selon la revendication précédente, **caractérisée par le fait que** la composition qu'elle est apte à former un dépôt ayant un indice de tenue supérieur ou égal à 50 %, voire supérieure à 60%, voire même supérieure ou égale à 65%.

**17.** Composition selon l'une quelconque des revendications précédentes, **caractérisée en ce qu'**elle comprend, en outre, une ou des matières colorantes choisies parmi les colorants, les pigments, les nacres et les paillettes.

**18.** Composition selon l'une quelconque des revendications précédentes, **caractérisée par le fait qu'**elle comprend en outre au moins un corps gras choisi parmi les cires, les corps gras pâteux, les huiles et leurs mélanges.

**19.** Composition selon l'une quelconque des revendications précédentes, **caractérisée par le fait qu'**elle comprend un ingrédient cosmétique choisi parmi les vitamines, les épaississants, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, les agents anti-chutes des cheveux, les agents de soin du cil, les agents antipelliculaires, les agents propulseurs, ou leurs mélanges.

**20.** Composition cosmétique selon l'une quelconque des revendications précédentes, **caractérisée en ce qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre, de stick ou de solide coulé.

**21.** Composition cosmétique selon l'une quelconque des revendications 1 à 20, **caractérisée en ce qu'**elle se présente sous forme anhydre.

**22.** Composition cosmétique selon l'une quelconque des revendications précédentes, **caractérisée en ce qu'**il s'agit d'une composition de maquillage ou de soin des matières kératiniques.

**23.** Composition selon l'une des revendications 1 à 22, **caractérisée en ce qu'**il s'agit d'un produit de maquillage des lèvres.

**24.** Composition cosmétique comprenant au moins un ester d'alcool alcoxylé et d'acide carboxylique et au moins un polymère filmogène non cristallin choisi parmi les polymères et copolymères vinyliques notamment acryliques et éthyléniques, les polymères et copolymères uréthanes, les polymères et copolymères polyesters, les polymères et copolymères polyamides, notamment les polyamides siliconés, les polymères et copolymères polyurées, les polymères et copolymères cellulosiques comme la nitrocellulose, les polymères et copolymères siliconés.

**25.** Utilisation cosmétique d'une composition selon l'une des revendications 1 à 24.

**26.** Procédé de maquillage et/ou de soin de la peau, des lèvres comprenant l'application sur la peau, les lèvres et/ou les phanères d'au moins une composition selon l'une quelconque des revendications 1 à 24.

Office européen des brevets

# RAPPORT DE RECHERCHE EUROPEENNE

Numéro de la demande
EP 05 29 2449

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,X | WO 2004/052076 A (SMITH, RONALD, J; SMITH, MARIA, K) 24 juin 2004 (2004-06-24)<br>* page 5, ligne 24 - page 6, ligne 9 * | 7-26 | INV.<br>A61K8/39<br>A61K8/81<br>A61Q1/06<br>A61Q19/00 |
| X | PUNTO, LOUIS L. ET AL: "Multifunctionality from a new polymeric ester"<br>COSMETICS & TOILETRIES , 119(11), 53-56, 58 CODEN: CTOIDG; ISSN: 0361-4387, novembre 2004 (2004-11), XP009066085<br>* page 58; tableau 1 * | 7,9-26 | |
| X | US 2004/185018 A1 (PATIL ANJALI ABHIMANYU [US] ET AL) 23 septembre 2004 (2004-09-23)<br>* exemples 1,2 *<br>* revendications *<br>* alinéas [0024] - [0042] *<br>* alinéas [0065] - [0068] * | 24-26 | |
| D,X | US 2002/192249 A1 (SMITH RONALD J ET AL) 19 décembre 2002 (2002-12-19)<br>* revendications *<br>* alinéa [0056]; exemples * | 24-26 | DOMAINES TECHNIQUES RECHERCHES (IPC) |
| | | | A61K<br>A61Q |
| X | US 2004/247543 A1 (HUERTA JOSE L ET AL) 9 décembre 2004 (2004-12-09)<br>* exemples 1,2 * | 24-26 | |
| D,X | WO 03/013439 A (CRODA, INC) 20 février 2003 (2003-02-20)<br>* revendications 60,72,88 *<br>* page 42, ligne 27 - page 45, ligne 2 *<br>* page 45, ligne 10 - page 46, ligne 5 * | 24-26 | |
| X | US 2003/069388 A1 (LAWSON NELSON E ET AL) 10 avril 2003 (2003-04-10)<br>* exemple 6 *<br>* revendications *<br>-/-- | 24-26 | |

Le présent rapport a été établi pour toutes les revendications

| Lieu de la recherche | Date d'achèvement de la recherche | Examinateur |
|---|---|---|
| Munich | 4 mai 2006 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

2

EPO FORM 1503 03.82 (P04C02)

Office européen des brevets

# RAPPORT DE RECHERCHE EUROPEENNE

Numéro de la demande
EP 05 29 2449

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 352 441 A (MAUSNER ET AL) 4 octobre 1994 (1994-10-04) * revendication 31 * * tableau 1 * * colonne 3, ligne 52 - colonne 4, ligne 34 * ----- | 24-26 | |
| X | WO 2004/066918 A (L'OREAL; ATIS, BALANDA) 12 août 2004 (2004-08-12) * revendications * * exemple 5 * * page 15, ligne 7 - page 21, ligne 21 * ----- | 24-26 | |
| X | US 6 423 324 B1 (MURPHY JOHN ET AL) 23 juillet 2002 (2002-07-23) * exemples 1-5,10-16 * * colonne 4, ligne 61 - colonne 5, ligne 7 * ----- | 24-26 | |
| X | US 2004/180032 A1 (MANELSKI JEAN MARIE ET AL) 16 septembre 2004 (2004-09-16) * exemple 1 * * revendications * * alinéas [0006] - [0010], [0030] * ----- | 24-26 | DOMAINES TECHNIQUES RECHERCHES (IPC) |
| X | US 2004/214749 A1 (PATT LEONARD M) 28 octobre 2004 (2004-10-28) * exemple 2 * ----- | 24-26 | |
| X | US 6 063 368 A (KAPSNER ET AL) 16 mai 2000 (2000-05-16) * revendications * * exemple * ----- | 24-26 | |

Le présent rapport a été établi pour toutes les revendications

| Lieu de la recherche | Date d'achèvement de la recherche | Examinateur |
|---|---|---|
| Munich | 4 mai 2006 | Hauss, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

2
EPO FORM 1503 03.82 (P04C02)

**ANNEXE AU RAPPORT DE RECHERCHE EUROPEENNE RELATIF A LA DEMANDE DE BREVET EUROPEEN NO.** EP 05 29 2449

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

04-05-2006

| Document brevet cité au rapport de recherche | Date de publication | Membre(s) de la famille de brevet(s) | Date de publication |
|---|---|---|---|
| WO 2004052076 A | 24-06-2004 | EP 1575532 A2 | 21-09-2005 |
| US 2004185018 A1 | 23-09-2004 | AUCUN | |
| US 2002192249 A1 | 19-12-2002 | US 2004210077 A1 | 21-10-2004 |
| US 2004247543 A1 | 09-12-2004 | BR 0212686 A | 19-10-2004 |
| | | CA 2461126 A1 | 03-04-2003 |
| | | GB 2396813 A | 07-07-2004 |
| | | MX PA04002645 A | 08-07-2004 |
| | | WO 03026595 A1 | 03-04-2003 |
| | | US 2003059383 A1 | 27-03-2003 |
| WO 03013439 A | 20-02-2003 | CN 1564675 A | 12-01-2005 |
| | | EP 1414397 A2 | 06-05-2004 |
| | | JP 2005511493 T | 28-04-2005 |
| US 2003069388 A1 | 10-04-2003 | AUCUN | |
| US 5352441 A | 04-10-1994 | AUCUN | |
| WO 2004066918 A | 12-08-2004 | EP 1592396 A2 | 09-11-2005 |
| US 6423324 B1 | 23-07-2002 | AU 7507401 A | 02-01-2002 |
| | | WO 0197758 A2 | 27-12-2001 |
| US 2004180032 A1 | 16-09-2004 | AUCUN | |
| US 2004214749 A1 | 28-10-2004 | AU 2004233865 A1 | 11-11-2004 |
| | | CA 2523341 A1 | 11-11-2004 |
| | | US 2005197282 A1 | 08-09-2005 |
| | | WO 2004096258 A1 | 11-11-2004 |
| US 6063368 A | 16-05-2000 | AUCUN | |

EPO FORM P0460

Pour tout renseignement concernant cette annexe : voir Journal Officiel de l'Office européen des brevets, No.12/82